(19) Europäisches Patentamt / European Patent Office / Office européen des brevets

(11) **EP 2 075 321 B1**

(12) **EUROPÄISCHE PATENTSCHRIFT**

(45) Veröffentlichungstag und Bekanntmachung des Hinweises auf die Patenterteilung:
**03.08.2011 Patentblatt 2011/31**

(51) Int Cl.:
*C11B 9/02* *(2006.01)*   *A23L 1/226* *(2006.01)*
*A23L 2/56* *(2006.01)*

(21) Anmeldenummer: **08171407.3**

(22) Anmeldetag: **11.12.2008**

(54) **Verfahren zur Herstellung eines Aromakonzentrates sowie ein Aromakonzentrat**

Method for manufacturing an aroma concentrate and aroma concentrate

Procédé destiné à la fabrication d'un concentré d'arômes et concentré d'arômes

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.12.2007 EP 07123386**

(43) Veröffentlichungstag der Anmeldung:
**01.07.2009 Patentblatt 2009/27**

(73) Patentinhaber: **Symrise AG**
**37603 Holzminden (DE)**

(72) Erfinder:
• **Reiß, Ingo**
**37603 Holzminden (DE)**
• **Erfurt, Harry**
**37170 Uslar (DE)**
• **Ott, Frank**
**37603 Holzminden (DE)**
• **Kindel, Günter**
**37671 Höxter (DE)**
• **Gabriel, Bernd**
**37643 Negenborn (DE)**

(74) Vertreter: **Eisenführ, Speiser & Partner**
**Am Kaffee-Quartier 3**
**28217 Bremen (DE)**

(56) Entgegenhaltungen:
**EP-A- 0 082 284     EP-A- 0 198 262**
**EP-A- 1 205 544     WO-A-02/36720**
**DE-A1- 19 835 542**

• **DATABASE WPI Week 199405 Thomson Scientific, London, GB; AN 1994-040123 XP002513759 & JP 05 345899 A (JUMOKU CHUSHUTSU SEIBUN RIYO) 27. Dezember 1993 (1993-12-27)**

**Beschreibung**

Die vorliegende Erfindung betrifft ein Verfahren zur Herstellung eines Aromakonzentrates, ein Aromakonzentrat, herstellbar nach einem erfindungsgemäßen Verfahren, Produkte umfassend ein oder mehrere erfindungsgemäße Aromakonzentrate, sowie Verfahren zur Anreicherung von Geschmacks- und/oder Duftstoffen.

Der immer steigende Bedarf an Produkten im Nahrungsmittel- und Getränkesektor erhöht auch den Bedarf an immer neuen Aromen bzw. Aromaprofilen.

Ein Aroma wird oftmals durch mehrere Geschmacks- und/oder Duftstoffe vermittelt, wobei sowohl Geschmacks- und/oder Duftstoffe, die einen positiven sensorischen Eindruck vermitteln, als auch Geschmacks- und/oder Duftstoffe, die einen negativen sensorischen Eindruck vermitteln, den sensorischen Eindruck beeinflussen.

In DE 19835542 A1 wird ein Verfahren zur Anreicherung, Trennung und Aufreinigung binärer Aromastoffgemische in Einzelverbindungen beschrieben. Hierbei entsteht einer der beiden korrespondierenden Stoffe dieses binären Stoffgemisches aus dem jeweils anderen und beide Stoffe werden über Zeolithe voneinander getrennt. Eine Aufspaltung eines komplexeren Gemisches ist nicht beschrieben.

DE 198 35 542 A1 betrifft ein Verfahren zur Gewinnung reiner Verbindungen aus wässrigen und/oder organischen Lösungen, durch Inkontaktbringen mit Zeolithen, Trennung der Zeolithe von der Ausgangslösung und Desorption des absorbierten Stoffes vom Zeolithen mit einem Lösungsmittel (Spalte 1, Zeilen 52 bis 67). Als abzutrennende Duftstoffe sind unter anderem Linalool, Geraniol, Menthon und Carvon genannt.

Es ist ein Ziel der vorliegenden Erfindung ein Verfahren bereitzustellen, durch das aus einem wässrigen Aroma ein Aromakonzentrat hergestellt werden kann, das ein oder mehrere Geschmacks- und/oder Duftstoffe, die einen positiven sensorischen Eindruck vermitteln, und ein oder mehrere Geschmacks- und/oder Duftstoffe, die einen negativen sensorischen Eindruck vermitteln, umfasst, wobei in dem resultierenden Aromakonzentrat der oder die Geschmacks- und/oder Duftstoffe, die einen positiven sensorischen Eindruck vermitteln, im Vergleich zu dem oder den Geschmacks- und/oder Duftstoffen, die einen negativen sensorischen Eindruck vermitteln, um Verhältnis höher angereichert vorliegen als in dem wässrigen Aroma.

Die vorliegende Problemstellung wird dementsprechend durch die Gegenstände der unabhängigen Ansprüche gelöst. Bevorzugte Ausgestaltungen werden insbesondere in den abhängigen Ansprüchen sowie der nachfolgenden Beschreibung dargestellt.

Demnach wird die vorliegende Problemstellung gelöst durch ein Verfahren gemäß Anspruch 1 zur Herstellung eines Aromakonzentrates, indem ein oder mehrere Geschmacks- und/oder Duftstoffe, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma höher angereichert sind, umfassend oder bestehend aus folgenden Schritten:

a) Bereitstellen eines wässrigen Aromas umfassend

   i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,

   ii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure,

b) Bereitstellen ein, zwei oder mehrerer lebensmittelgeeigneter Lösungsmittel,

c) Zugabe des oder der Lösungsmittel aus Schritt b) zu der Lösung aus Schritt a), wobei der resultierende Lösungsmittelanteil so eingestellt wird, dass die ein oder mehrere Geschmacks- und/oder Duftstoffe aus der Gruppe i) unabhängig voneinander, jeweils einen log Pow*-Wert von größer oder gleich 1,20 aufweisen und der log Pow*-Wert wie folgt berechnet wird:

$$\log Pow^* = \log Pow - x * P'(x) / \log Pow$$

mit log Pow als dem dekadischen Logarithmus des Verteilungskoeffizienten des Geschmacks- und/oder Duftstoffes zwischen 1-Octanol zu Wasser,
mit x als dem resultierenden Lösungsmittelanteil bezogen auf den Gesamtvolumenanteil, als 1,0 normiert und
mit P'(x) als Polaritätsparameter für das entsprechende Lösungsmittel bei dem resultierenden Lösungsmittelanteil x,

d) Bereitstellen eines Adsorptionsmaterials in einer geeigneten Vorrichtung,

e) Durchleiten der Lösung aus Schritt c) durch eine Vorrichtung mit Adsorptionsmaterial aus Schritt d), so dass vorwiegend die ein oder mehreren Geschmacks- und/oder Duftstoffe, die einen positiven sensorischen Eindruck vermitteln, an das Absorptionsmaterial adsorbiert werden,

f) Bereitstellen von ein, zwei oder mehreren lebensmittelgeeigneten Lösungsmitteln und

g) Desorbieren der Geschmacks- und/oder Duftstoffe von dem Adsorptionsmaterial aus Schritt e) mit dem oder den Lösungsmitteln aus Schritt f), so dass in dem resultierenden Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a).

Ein dementsprechendes Verfahren wird im Rahmen dieser Erfindung auch "Adsorptionsverfahren" genannt.
Ein weiterer Gegenstand der vorliegenden Erfindung betrifft insbesondere ein Verfahren gemäß Anspruch 8 zur Herstellung eines Aromakonzentrates, in dem ein oder mehrere Geschmacks- und/oder Duftstoffe, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma höher angereichert sind, umfassend oder bestehend aus folgenden Schritten:

a) Bereitstellen eines wässrigen Aromas umfassend

ii. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,

iii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure,

wobei der Lösungsmittelanteil bezogen auf das Gesamtvolumen der wässrigen Lösung im Bereich von 0 Vol.-% bis kleiner 1 Vol.-% liegt,

b) Bereitstellen eines Adsorptionsmaterials in einer geeigneten Vorrichtung,

c) Durchleitung der Lösung aus Schritt a) durch die Vorrichtung mit Adsorptionsmaterial aus Schritt b), so dass sowohl die ein oder mehreren Geschmacks- und/oder Duftstoffe aus der Gruppe i) als auch die ein oder mehreren Geschmacks- und/oder Duftstoffe aus der Gruppe ii) an das Absorptionsmaterial adsorbiert werden,

d) Bereitstellen eines oder mehrerer Lösungsmittel oder Lösungsmittelgemische mit Wasser, wobei die Lösungsmittel oder die Lösungsmittelgemische so gewählt werden, dass ein oder mehrere Geschmacks- und/oder Duftstoffe der Gruppe ii) einen log Pow*-Wert von 1,20 oder kleiner aufweisen und

e) Desorbieren der Geschmacks- und/oder Duftstoffe von dem Adsorptionsmaterial aus Schritt c) mit den Lösungsmitteln oder den Lösungsmittelgemischen aus Schritt d), wobei ein, zwei, drei, vier, fünf, sechs, sieben oder mehrere Fraktionen über die Zeit gesammelt werden und eine dieser Fraktion oder eine Mischung aus zwei, drei, vier, fünf, sechs, sieben oder mehreren dieser Fraktionen das Aromakonzentrat bilden, wobei in dem resultierenden Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a), wobei das zur Desorption verwendete oder die zur Desorption verwendeten Lösungsmittel so gewählt werden, dass die zu desorbierenden Geschmacks- und/oder Duftstoffe einen log Pow* Wert von 1,20 oder kleiner besitzen und der log Pow*-Wert wie folgt berechnet wird:

$$\log \text{Pow}^* = \log \text{Pow} - x * P'(x) / \log \text{Pow}$$

mit log Pow als dem dekadischen Logarithmus des Verteilungskoeffizienten des Geschmacks- und/oder Duftstoffes zwischen 1-Octanol zu Wasser,

mit x als dem resultierenden Lösungsmittelanteil bezogen auf den Gesamtvolumenanteil, als 1,0 normiert und

mit P'(x) als Polaritätsparameter für das entsprechende Lösungsmittel bei dem resultierenden Lösungsmittelanteil x.

Ein dementsprechendes Verfahren wird im Rahmen dieser Erfindung auch "Desorptionsverfahren" genannt.

Ein weiterer Gegenstand der vorliegenden Erfindung betrifft ein Aromakonzentrat, herstellbar nach einem erfindungsgemäßen Herstellungsverfahren, wobei im Schritt a) des Verfahrens ein spezielles wässriges Aroma bereit gestellt wird.

Des Weiteren betrifft ein Gegenstand der vorliegenden Erfindung Nahrungsmittel-, Genussmittel-, Getränke-, Halbfertig-, Mundhygiene-, kosmetische oder pharmazeutische Produkte umfassend ein oder mehrere erfindungsgemäße Aromakonzentrate sowie ein oder mehrere Grund-, Hilfs- und/oder Zusatzstoffe.

Des Weiteren betrifft ein Gegenstand der vorliegenden Erfindung die Verwendung von erfindungsgemäßen Aromakonzentraten in Nahrungsmittel-, Genussmittel-, Getränke-, Halbfertig-, Mundhygiene-, kosmetischen oder pharmazeutischen Produkten.

Ein weiterer Gegenstand der vorliegenden Erfindung betrifft die Verwendung erfindungsgemäßer Aromakonzentrate zur Herstellung von Nahrungsmittel-, Genussmittel-, Getränke-, Halbfertig-, Mundhygiene-, kosmetischen oder pharmazeutischen Produkten.

Zudem betrifft die vorliegende Erfindung ein Verfahren zur Anreicherung von ein oder mehreren Geschmacks- und/oder Duftstoffen, die einen positiven sensorischen Eindruck vermitteln, (z.B. in einem resultierenden Aromakonzentrat) unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma, umfassend oder bestehend aus folgenden Schritten:

a) Bereitstellen eines wässrigen Aromas umfassend

i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,

ii. ein oder mehrere Geschmacks- und/oder Duftstoffe, die einen negativen sensorischen Eindruck vermitteln, ausgewählt aus der Liste bestehend aus $C_1$-$C_5$Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure,

b) Bereitstellen ein, zwei oder mehrerer lebensmittelgeeigneter Lösungsmittel,

c) Zugabe des oder der Lösungsmittel aus Schritt b) zu der Lösung aus Schritt a), wobei der resultierende Lösungsmittelanteil so eingestellt wird, dass die ein oder mehrere Geschmacks- und/oder Duftstoffe aus der Gruppe i) unabhängig voneinander, jeweils einen log Pow*-Wert von größer oder gleich 1,20 aufweisen und der log Pow*-Wert wie folgt berechnet wird:

$$\log Pow^* = \log Pow - x * P'(x) / \log Pow$$

mit log Pow als dem dekadischen Logarithmus des Verteilungskoeffizienten des Geschmacks- und/oder Duftstoffes zwischen 1-Octanol zu Wasser,
mit x als dem resultierenden Lösungsmittelanteil bezogen auf den Gesamtvolumenanteil, als 1,0 normiert und
mit P'(x) als Polaritätsparameter für das entsprechende Lösungsmittel bei dem resultierenden Lösungsmittelanteil x,

d) Bereitstellen eines Adsorptionsmaterials in einer geeigneten Vorrichtung,

e) Durchleiten der Lösung aus Schritt c) durch eine Vorrichtung mit Adsorptionsmaterial aus Schritt d), so dass vorwiegend die ein oder mehreren Geschmacks- und/oder Duftstoffe, die einen positiven sensorischen Eindruck vermitteln, an das Absorptionsmaterial adsorbiert werden,

f) Bereitstellen von ein, zwei oder mehreren lebensmittelgeeigneten Lösungsmitteln und

g) Desorbieren der Geschmacks- und/oder Duftstoffe von dem Adsorptionsmaterial aus Schritt e) mit dem oder den Lösungsmitteln aus Schritt f), so dass (z,B. in dem resultierenden Aromakonzentrat) die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a).

Alternativ betrifft ein weiterer Gegenstand der vorliegenden Erfindung ein Verfahren zur Anreicherung von ein oder mehreren Geschmacks- und/oder Duftstoffen, die einen positiven sensorischen Eindruck vermitteln, (z.B. in einem resultierenden Aromakonzentrat) unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma, umfassend oder bestehend aus folgenden Schritten:

a) Bereitstellen eines wässrigen Aromas umfassend

i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat. Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine. Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,

ii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure, wobei der Lösungsmittelanteil bezogen auf das Gesamtvolumen der wässrigen Lösung im Bereich von 0 Vol.-% bis kleiner 1 Vol.-% liegt,

b) Bereitstellen eines Adsorptionsmaterials in einer geeigneten Vorrichtung,

c) Durchleitung der Lösung aus Schritt a) durch die Vorrichtung mit Adsorptionsmaterial aus Schritt b), so dass sowohl die ein oder mehreren Geschmacks- und/oder Duftstoffe aus der Gruppe i) als auch die ein oder mehreren Geschmacks- und/oder Duftstoffe aus der Gruppe ii) an das Absorptionsmaterial adsorbiert werden,

d) Bereitstellen eines oder mehrerer Lösungsmittel oder Lösungsmittelgemische mit Wasser, wobei die Lösungsmittel oder die Lösungsmittelgemische so gewählt werden, dass ein oder mehrere Geschmacks- und/oder Duftstoffe der Gruppe ii) einen log Pow*-Wert von 1,20 oder kleiner aufweisen und

e) Desorbieren der Geschmacks- und/oder Duftstoffe von dem Adsorptionsmaterial aus Schritt c) mit den Lösungsmitteln oder den Lösungsmittelgemischen aus Schritt d), wobei ein, zwei, drei, vier, fünf, sechs, sieben oder mehrere Fraktionen über die Zeit gesammelt werden und in einer dieser Fraktionen oder in einer Mischung aus zwei, drei, vier, fünf, sechs, sieben oder mehreren dieser Fraktionen die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a), wobei das zur Desorption verwendete oder die zur Desorption verwendeten Lösungsmittel so gewählt werden, dass die zu desorbierenden Geschmacks- und/oder Duftstoffe einen log Pow* Wert von 1,20 oder kleiner besitzen und der log Pow*-Wert wie folgt berechnet wird:

$$\log Pow^* = \log Pow - x \cdot P'(x) / \log Pow$$

mit log Pow als dem dekadischen Logarithmus des Verteilungskoeffizienten des Geschmacks- und/oder Duftstoffes zwischen 1-Octanol zu Wasser,
mit x als dem resultierenden Lösungsmittelanteil bezogen auf den Gesamtvolumenanteil, als 1,0 normiert und
mit P'(x) als Polaritätsparameter für das entsprechende Lösungsmittel bei dem resultierenden Lösungsmittelanteil x.

Die Gegenstände der vorliegenden Erfindung beruhen auf der Erkenntnis, dass mit den nachfolgenden Formeln 1 und 2 der Lösungsmittelanteil in einem wässrigen Aroma im Schritt c) des erfindungsgemäßen Adsorptionsverfahrens oder der Lösungsmittelanteil im Desorptionsmittel in Schritt e) des erfindungsgemäßen Desorptionsverfahrens eingestellt werden können, um eine erfindungsgemäße Anreicherung des oder der Geschmacks- und/oder Duftstoffe aus der Gruppe i) im Vergleich zu dem oder den Geschmacks- und/oder Duftstoffen aus der Gruppe ii) unabhängig voneinander jeweils um den Faktor 1,5 oder mehr bezogen auf das wässrige Aroma aus Schritt a) zu erreichen.

Formel 1:

$$P'(x) = P(Wasser) \times P(L\ddot{o}Mi) / (P(L\ddot{o}Mi) - x \cdot (P(L\ddot{o}Mi) - P(Wasser)))$$

mit P(Wasser) als dem Polaritätsparameter für Wasser, bestimmt nach Snyder und offenbart in "Flüssigkeitschromatographie HPLC - Theorie und Praxis" Günter J. Eppert, Friedr. Vieweg & Sohn Verlagsgesellschaft mbH, Braunschweig / Wiesbaden 1997, S. 84 und
mit P(LöMi) als den Polaritätsparametern für die entsprechenden Lösungsmittel, die ebenfalls in vorstehend genanntem Buch offenbart werden.

Formel 2:

$$\log Pow^* = \log Pow - x \cdot P'(x) / \log Pow$$

mit log Pow als dem dekadischen Logarithmus des Verteilungskoeffizienten einer Substanz zwischen 1-Octanol und Wasser,
mit x als dem resultierenden Lösungsmittelanteil bezogen auf den Gesamtvolumenanteil, als 1,0 normiert
und mit P'(x) als Polaritätsparameter für das entsprechende Lösungsmittel bei dem Lösungsmittelanteil x,
[0019]   Die Erfinder haben überraschenderweise gefunden, das der log Pow*-Wert für die jeweiligen Geschmacks- und/oder Duftstoffe eine Voraussage ermöglicht, in welchem Verhältnis der jeweilige Geschmacks- und/oder Duftstoff bei einem vorgegebenen Lösungsmittelanteil in der wässrigen Phase an das Adsorptionsmaterial adsorbiert oder in der wässrigen Phase verbleibt.

Die Theorie der Verteilung eines Stoffes zwischen 1-Octanol und Wasser besagt, dass bei einem log Pow-Wert von 0,0 ein Anteil von 50 % des isoliert betrachteten Stoffes in 1-Octanol und ein Anteil von 50 % in Wasser gelöst ist.

Die durch die vorstehenden mathematischen Formeln berechneten log Pow*-Werte geben Indikatoren wieder, wie sich ein oder mehrere Geschmacks- und/oder Duftstoffe zwischen dem Adsorptionsmaterial (entspricht einem angenommenen Pendant zu 1-Octanol) und Wasser verteilen.

Die vorstehenden mathematischen Formeln können zwar die vorhandenen komplexen Wechselwirkungen zwischen den Geschmacks- und/oder Duftstoffen selbst sowie zum verwendeten Adsorptionsmaterial lediglich annäherungsweise wiedergeben. Jedoch konnten vielfältige experimentelle Versuche in Bezug auf die vorliegende Erfindung bestätigen, dass ein erfindungsgemäßes Aromakonzentrat, in dem die ein oder mehreren Geschmacks- und/oder Duftstoffe aus der Gruppe i), die einen positiven sensorischen Eindruck vermitteln, in dem resultierenden Aromakonzentrat unabhängig voneinander, jeweils um den Faktor 1,5 oder mehr im Vergleich zu den ein oder mehreren Geschmacks- und/oder Duftstoffen aus der Gruppe ii), die einen negativen sensorischen Eindruck vermitteln, bezogen auf das wässrige Aroma, höher angereichert sind, wenn während des Adsorptionsvorgangs des erfindungsgemäßen Adsorptionsverfahrens der Anteil an einem oder mehreren Lösungsmitteln so eingestellt wird, dass die log Pow*-Werte des oder der Geschmacks- und/der Duftstoffe aus der Gruppe i) unabhängig voneinander einen log Pow*-Wert von größer oder gleich 1,20 aufweisen. In einer bevorzugten Ausgestaltung des erfindungsgemäßen Adsorptionsverfahrens wird der Anteil an Lösungsmittel in Schritt c) gleichzeitig so gewählt, dass ein oder mehrere Geschmacks- und/oder Duftstoffe der Gruppe ii), die einen positiven sensorischen Eindruck vermitteln, einen log Pow*-Wert von kleiner 1,20, bevorzugt 1,1 oder weniger, weiter bevorzugt bei 1,0 oder haben.

Für das erfindungsgemäße Desorptionsverfahren wurde gefunden, dass ein erfindungsgemäßes Aromakonzentrat hergestellt werden kann, wenn der Anteil eines oder mehrerer Lösungsmittel oder Lösungsmittelgemische mit Wasser während der Desorption so gewählt wird, dass ein oder mehrere an das Adsorptionsmaterial adsorbierte Geschmacks- und/oder Duftstoffe der Gruppe ii), die einen negativen sensorischen Eindruck vermitteln, einen log Pow*-Wert von 1,20 oder kleiner, bevorzugt einen log Pow*-Wert von 1,15 oder kleiner, weiter bevorzugt einen log Pow*-Wert von 1,0 oder kleiner haben, so dass ein, zwei, drei, vier, fünf, sechs, sieben oder mehrere Fraktionen über die Zeit gesammelt werden und eine dieser Fraktion oder eine Mischung aus zwei, drei, vier, fünf, sechs, sieben oder mehreren dieser Fraktionen das erfindungsgemäße Aromakonzentrat bilden, wobei das resultierende Aromakonzentrat dadurch gekennzeichnet ist, dass die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem ursprünglich verwendeten wässrigen Aroma. In einer bevorzugten Ausgestaltung des erfindungsgemäßen Desorptionsverfahrens sind die Lösungsmittel bzw. Lösungsmittelgemische in Schritt d) gleichzeitig so gewählt, dass ein oder mehrere Geschmacks- und/oder Duftstoffe der Gruppe i), die einen positiven sensorischen Eindruck vermitteln, einen log Pow*-Wert von 1,20 oder größer, bevorzugt, 1,30 oder größer, weiter bevorzugt 1,50 oder größer haben.

Der Lösungsmittelanteil, wie vorstehend beschrieben, kann während des Desorptionsvorgangs des erfindungsgemäßen Desorptionsverfahrens auch variiert werden, indem der Lösungsmittelanteil über die Zeit des Desorptionsvorgangs kontinuierlich oder diskontinuierlich ansteigt (Lösungsmittelgradient), damit ein, zwei, drei, vier, fünf, sechs, sieben oder mehrere Fraktionen über die Zeit gesammelt werden und eine dieser Fraktionen oder eine Mischung aus zwei, drei, vier, fünf, sechs, sieben oder mehreren dieser Fraktionen das erfindungsgemäße Aromakonzentrat bilden, wobei in dem resultierenden Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma.

Die nachfolgende Tabelle 1 stellt die Polaritätsparameter (P bzw. P' für die Lösungsmittelgemische) an ausgewählten erfindungsgemäßen Lösungsmitteln, nämlich n-Propanol, Ethanol, Ethylacetat, Methanol und Wasser, dar.

Tabelle 1:

| Substanz | x P | 0,02 P' | 0,04 P' | 0,06 P' | 0,08 P' | 0,1 P' | 0,2 P' |
|---|---|---|---|---|---|---|---|
| n-Propanol | 4 | 9,893 | 9,605 | 9,332 | 9,075 | 8,831 | 7,786 |
| Ethanol | 4,3 | 9,928 | 9,669 | 9,424 | 9,191 | 8,969 | 8,004 |
| Ethylacetat | 4,4 | 9,938 | 9,689 | 9,452 | 9,227 | 9,012 | 8,072 |
| Methanol | 5,1 | 10,000 | 9,808 | 9,623 | 9,444 | 9,273 | 8,500 |
| Wasser | 10,2 | | | | | | |

| Substanz | 0,3 P' | 0,4 P' | 0,5 P' | 0,6 P' | 0,7 P' | 0,8 P' | 0,9 P' | 1 P' |
|---|---|---|---|---|---|---|---|---|
| n-Propanol | 6,962 | 6,296 | 5,746 | 5,285 | 4,892 | 4,554 | 4,259 | 4,000 |
| Ethanol | 7,226 | 6,586 | 6,050 | 5,594 | 5,203 | 4,863 | 4,564 | 4,300 |
| Ethylacetat | 7,309 | 6,679 | 6,148 | 5,695 | 5,305 | 4,965 | 4,665 | 4,400 |
| Methanol | 7,846 | 7,286 | 6,800 | 6,375 | 6,000 | 5,667 | 5,368 | 5,100 |
| Wasser | | | | | | | | |

In der nachfolgenden Tabelle 2 wird der Einfluss des in Tabelle 1 auf Basis der vorstehenden Formel 1 errechneten Polaritätsparameters auf den log Pow-Wert verschiedener sensorisch wirksamer Geschmacks- und/oder Duftstoffe anhand der vorstehend beschriebenen Formel 2 dargestellt.

Tabelle 2:

| Substanz | log Pow | Solvens | P' 2% | P' 4% | P' 6% | P' 8% | P' 10% |
|---|---|---|---|---|---|---|---|
| Diacetyl | -1,33 | Ethanol | -1,48 | -1,62 | -1,76 | -1,88 | -2,00 |
| Furfurylalkohol | 0,28 | Ethanol | -0,43 | -1,10 | -1,74 | -2,35 | -2,92 |
| Methylmercaptan | 0,65 | Ethanol | 0,34 | 0,05 | -0,22 | -0,48 | -0,73 |
| Furfural | 0,73 | Ethanol | 0,46 | 0,20 | -0,04 | -0,28 | -0,50 |
| Butanol | 0,88 | Ethanol | 0,65 | 0,44 | 0,24 | 0,04 | -0,14 |
| 3-Mebutanol | 1,28 | Ethanol | 1,12 | 0,98 | 0,84 | 0,71 | 0,58 |
| 2-Mebutanol | 1,29 | Ethanol | 1,14 | 0,99 | 0,85 | 0,72 | 0,59 |
| Phenol | 1,48 | Ethanol | 1,35 | 1,22 | 1,10 | 0,98 | 0,87 |
| Linalooloxid | 1,56 | Ethanol | 1,43 | 1,31 | 1,20 | 1,09 | 0,99 |
| Ethyldimethylpyrazin | 1,60 | Ethanol | 1,48 | 1,36 | 1,25 | 1,14 | 1,04 |
| 2E-Hexenal | 1,61 | Ethanol | 1,49 | 1,37 | 1,26 | 1,15 | 1,05 |
| Benzaldehyd | 1,64 | Ethanol | 1,52 | 1,40 | 1,30 | 1,19 | 1,09 |
| 2E-Hexenol | 1,75 | Ethanol | 1,64 | 1,53 | 1,43 | 1,33 | 1,24 |
| Butylacetat | 1,77 | Ethanol | 1,66 | 1,55 | 1,45 | 1,35 | 1,26 |
| Ethylbutyrat | 1,77 | Ethanol | 1,66 | 1,55 | 1,45 | 1,35 | 1,26 |
| Furfurylthiol-2 | 1,90 | Ethanol | 1,80 | 1,70 | 1,60 | 1,51 | 1,43 |
| Hexanal | 1,97 | Ethanol | 1,87 | 1,77 | 1,68 | 1,60 | 1,51 |
| Isopropylmethoxypyrazin | 2,09 | Ethanol | 1,99 | 1,90 | 1,82 | 1,74 | 1,66 |
| Methyljasmonat | 2,12 | Ethanol | 2,03 | 1,94 | 1,85 | 1,77 | 1,70 |
| Indol | 2,14 | Ethanol | 2,05 | 1,96 | 1,88 | 1,80 | 1,72 |
| Methylcapronat | 2,30 | Ethanol | 2,21 | 2,13 | 2,05 | 1,98 | 1,91 |
| Ethyl methylbutyrat-2 | 2,38 | Ethanol | 2,30 | 2,22 | 2,14 | 2,07 | 2,00 |
| gamma-Decalacton | 2,38 | Ethanol | 2,30 | 2,22 | 2,14 | 2,07 | 2,00 |
| delta Decalacton | 2,42 | Ethanol | 2,34 | 2,26 | 2,19 | 2,12 | 2,05 |
| Jasminlacton | 2,47 | Ethanol | 2,39 | 2,31 | 2,24 | 2,17 | 2,11 |
| Vinylguaiacol | 2,48 | Ethanol | 2,40 | 2,32 | 2,25 | 2,18 | 2,12 |
| Hexylacetat | 2,83 | Ethanol | 2,76 | 2,69 | 2,63 | 2,57 | 2,51 |
| Methylpropyldisulfid | 2,83 | Ethanol | 2,76 | 2,69 | 2,63 | 2,57 | 2,51 |
| Octanal | 3,03 | Ethanol | 2,96 | 2,90 | 2,84 | 2,79 | 2,73 |
| Citral | 3,17 | Ethanol | 3,11 | 3,05 | 2,99 | 2,94 | 2,89 |
| Linalool | 3,28 | Ethanol | 3,22 | 3,16 | 3,11 | 3,06 | 3,01 |
| beta-Ionon | 3,85 | Ethanol | 3,80 | 3,75 | 3,70 | 3,66 | 3,62 |
| alpha-Ionon | 3,86 | Ethanol | 3,81 | 3,76 | 3,71 | 3,67 | 3,63 |
| Dipropyldisulfid | 3,90 | Ethanol | 3,85 | 3,80 | 3,76 | 3,71 | 3,67 |
| p-Menthenthiol-1,8 | 4,04 | Ethanol | 3,99 | 3,94 | 3,90 | 3,86 | 3,82 |
| Nootkaton | 4,10 | Ethanol | 4,05 | 4,01 | 3,96 | 3,92 | 3,88 |

| beta-Damascenon | 4,40 | Ethanol | 4,35 | 4,31 | 4,27 | 4,23 | 4,20 |
|---|---|---|---|---|---|---|---|
| Dipropyltrisulfid | 5,05 | Ethanol | 5,01 | 4,97 | 4,94 | 4,90 | 4,87 |

| Substanz | P' 30% | P' 40% | P 50% | P' 60% | P' 70% | P' 80% | P' 90% | P' 100% |
|---|---|---|---|---|---|---|---|---|
| Diacetyl | -2,96 | -3,31 | -3,60 | -3,85 | -4,07 | -4,25 | -4,42 | -4,56 |
| Furfurylalkohol | -7,46 | -9,13 | -10,52 | -11,71 | -12,73 | -13,61 | -14,39 | -15,08 |
| Methylmercaptan | -2,68 | -3,40 | -4,00 | -4,51 | -4,95 | -5,33 | -5,67 | -5,97 |
| Furfural | -2,24 | -2,88 | -3,41 | -3,87 | -4,26 | -4,60 | -4,90 | -5,16 |
| Butanol | -1,58 | -2,11 | -2,56 | -2,93 | -3,26 | -3,54 | -3,79 | -4,01 |
| 3-Methylbutanol | -0,41 | -0,78 | -1,08 | -1,34 | -1,57 | -1,76 | -1,93 | -2,08 |
| 2-Methylbutanol | -0,39 | -0,75 | -1,05 | -1,31 | -1,53 | -1,73 | -1,89 | -2,04 |
| Phenol | 0,02 | -0,30 | -0,56 | -0,79 | -0,98 | -1,15 | -1,30 | -1,43 |
| Linalooloxid | 0,17 | -0,13 | -0,38 | -0,59 | -0,77 | -0,93 | -1,07 | -1,20 |
| Ethyldimethylpyrazin | 0,25 | -0,05 | -0,29 | -0,50 | -0,68 | -0,83 | -0,97 | -1,09 |
| 2E-Hexenal | 0,26 | -0,03 | -0,27 | -0,47 | -0,65 | -0,81 | -0,94 | -1,06 |
| Benzaldehyd | 0,32 | 0,03 | -0,20 | -0,41 | -0,58 | -0,73 | -0,86 | -0,98 |
| 2E-Hexenol | 0,51 | 0,24 | 0,02 | -0,17 | -0,33 | -0,47 | -0,60 | -0,71 |
| Butylacetat | 0,55 | 0,28 | 0,06 | -0,13 | -0,29 | -0,43 | -0,55 | -0,66 |
| Ethylbutyrat | 0,55 | 0,28 | 0,06 | -0,13 | -0,29 | -0,43 | -0,55 | -0,66 |
| Furfurylthiol-2 | 0,76 | 0,51 | 0,31 | 0,13 | -0,02 | -0,15 | -0,26 | -0,36 |
| Hexanal | 0,87 | 0,63 | 0,43 | 0,27 | 0,12 | 0,00 | -0,12 | -0,21 |
| Isopropylmethoxypyrazin | 1,05 | 0,83 | 0,64 | 0,48 | 0,35 | 0,23 | 0,12 | 0,03 |
| Methyljasmonat | 1,10 | 0,88 | 0,69 | 0,54 | 0,40 | 0,29 | 0,18 | 0,09 |
| Indol | 1,13 | 0,91 | 0,73 | 0,57 | 0,44 | 0,32 | 0,22 | 0,13 |
| Methylcapronat | 1,36 | 1,15 | 0,98 | 0,84 | 0,72 | 0,61 | 0,51 | 0,43 |
| Ethyl methylbutyrat-2 | 1,47 | 1,27 | 1,11 | 0,97 | 0,85 | 0,75 | 0,65 | 0,57 |
| gamma-Decalacton | 1,47 | 1,27 | 1,11 | 0,97 | 0,85 | 0,75 | 0,65 | 0,57 |
| delta Decalacton | 1,52 | 1,33 | 1,17 | 1,03 | 0,92 | 0,81 | 0,72 | 0,64 |
| Jasminlacton | 1,59 | 1,40 | 1,25 | 1,11 | 1,00 | 0,90 | 0,81 | 0,73 |
| Vinylguaiacol | 1,61 | 1,42 | 1,26 | 1,13 | 1,01 | 0,91 | 0,82 | 0,75 |
| Hexylacetat | 2,06 | 1,90 | 1,76 | 1,64 | 1,54 | 1,46 | 1,38 | 1,31 |
| Methylpropyldisulfid | 2,06 | 1,90 | 1,76 | 1,64 | 1,54 | 1,46 | 1,38 | 1,31 |
| Octanal | 2,31 | 2,16 | 2,03 | 1,92 | 1,83 | 1,75 | 1,67 | 1,61 |
| Citral | 2,49 | 2,34 | 2,22 | 2,11 | 2,02 | 1,94 | 1,87 | 1,81 |
| Linalool | 2,62 | 2,48 | 2,36 | 2,26 | 2,17 | 2,09 | 2,03 | 1,97 |
| beta-Ionon | 3,29 | 3,17 | 3,06 | 2,98 | 2,90 | 2,84 | 2,78 | 2,73 |
| alpha-Ionon | 3,30 | 3,18 | 3,08 | 2,99 | 2,92 | 2,85 | 2,80 | 2,75 |
| Dipropyldisulfid | 3,34 | 3,22 | 3,12 | 3,04 | 2,97 | 2,90 | 2,85 | 2,80 |

| | | | | | | | | |
|---|---|---|---|---|---|---|---|---|
| p-Menthenthiol-1,8 | 3,50 | 3,39 | 3,29 | 3,21 | 3,14 | 3,08 | 3,02 | 2,98 |
| Nootkaton | 3,57 | 3,46 | 3,36 | 3,28 | 3,21 | 3,15 | 3,10 | 3,05 |
| beta-Damascenon | 3,91 | 3,80 | 3,71 | 3,64 | 3,57 | 3,52 | 3,47 | 3,42 |
| Dipropyltrisulfid | 4,62 | 4,53 | 4,45 | 4,39 | 4,33 | 4,28 | 4,24 | 4,20 |

Anhand der Tabelle 2 ist zu erkennen, dass je höher der Lösungsmittelanteil während des Adsorptionsvorgangs wird, die log-Pow*-Werte grundsätzlich sinken, d.h. dass der Anteil an Geschmacks- und/oder Duftstoff, der in dem wässrigen Aroma verbleibt und nicht an das Adsorptionsmittel adsorbiert, steigt. Das hoch polare Diacetyl besitzt eine sehr hohe Affinität zu Wasser. Durch Zusatz von einem Lösungsmittel zu Wasser erhöht man zu einem kleinen Grad den unpolaren Charakter der Mischung. Daher werden zwar die log Pow* Werte des Diacetyl auch negativer, aber nicht so stark ansteigend wie die log Pow* Werte derjenigen Substanzen, die einen positiven log Pow Wert besitzen.

Gemäß der vorliegenden Erfindung bedeutet der Begriff "wässriges Aroma" eine Lösung, Emulsion oder Suspension, die einen Wasseranteil von mindestens 95% bezogen auf das Gesamtvolumen des wässrigen Aromas besitzt, wobei das wässrige Aroma ein oder mehrere der Geschmacks- und/oder Duftstoffe aus der Gruppe i) sowie ein oder mehrere Geschmacks- und/oder Duftstoffe aus der Gruppe ii) umfassen. Die Geschmacks- und/oder Duftstoffe liegen dabei üblicherweise in einer Konzentration von 0.001 bis 1000 ppm bezogen auf das Gesamtvolumen des wässrigen Aromas vor. Die wässrigen Aromen, die im Schritt a) des erfindungsgemäßen Absorptions- bzw. Desorptionsverfahren bereit gestellt werden, können natürlichen, sowie synthetischen Ursprungs sein.

Gemäß der vorliegenden Erfindung bedeutet der Begriff "Aromakonzentrat" eine Lösung, Emulsion oder Suspension, die ein oder mehrere der Geschmacks- und/oder Duftstoffe aus der Gruppe i) unabhängig voneinander, jeweils um den Faktor 1,50 oder mehr im Vergleich zu ein oder mehreren Geschmacks- und/oder Duftstoffen aus der Gruppe ii) bezogen auf das wässrige Aroma höher angereichert umfasst. Der Anteil an Geschmacks- und/oder Duftstoffe liegt dabei üblicherweise im Bereich 0,1 bis 20%, bevorzugt 1 bis 15%, weiter bevorzugt im Bereich von 2 bis 10%. Das erfindungsgemäße Aromakonzentrat kann durch das Verfahren bedingt Wasser enthalten kann mit weiteren Anteilen an Wasser oder anderen Lösungsmitteln verdünnt werden oder kann durch Abdestillieren des Gemisches, Wasser und/oder Lösungsmittel enthaltend, mittels üblicher Verfahren ganz oder teilweise aufkonzentriert werden. Erfindungsgemäße Aromakonzentrate werden üblicherweise in erfindungsgemäßen Produkten, insbesondere Nahrungsmittel-, Genussmittel-, Getränke-, Halbfertig-, Mundhygiene-, kosmetische oder pharmazeutische Produkte als Top-Note-Flavourings verwendet.

Im Rahmen der vorliegenden Erfindung bedeutet das Anreicherungsverhältnis, wonach "die ein oder mehreren Geschmacks- und/oder Duftstoffe aus der Gruppe i) in dem resultierenden Aromakonzentrat unabhängig voneinander, jeweils um den Faktor 1,5 oder mehr im Vergleich zu ein oder mehreren Geschmacks- und/oder Duftstoffen aus der Gruppe ii) bezogen auf das wässrige Aroma höher angereichert vorliegen", dass in dem wässrigen Aroma der Anteil an ein oder mehreren Geschmacks- und/oder Duftstoffen aus der Gruppe ii) im Vergleich zu ein oder mehreren Geschmacks- und/oder Duftstoffen aus der Gruppe i) annähernd gleich oder höher liegt als in dem erfindungsgemäßen Aromakonzentrat.

Dies wird beispielhaft an den Werten der nachfolgenden Tabelle 3 gezeigt, worin die Konzentrationen verschiedener üblicher Inhaltsstoffe einer wässrigen Orangenlösung sowie ein daraus resultierendes erfindungsgemäßes Aromakonzentrat dargestellt werden. Das erfindungsgemäße Aromakonzentrat wurde erfindungsgemäß nach dem Adsorptionsverfahren erhalten, indem das wässrige Aroma so eingestellt wurde, dass sie einen Volumenanteil von 2% Ethanol aufwies (entspricht Schritt c) des erfindungsgemäßen Adsorptionsverfahrens) und durch eine Vorrichtung mit Adsorptionsmaterial gemäß dem vorliegenden erfindungsgemäßen Verfahrens durchgeleitet wurde. Desorbiert wurde mit reinem, unvergälltem Ethanol (96,5 Vol.%).

Tabelle 3:

| | "wässriges Aroma" | "Aromakonzentrat" | Anreicherung |
|---|---|---|---|
| Name MI | [ppm] | [ppm] | [-fach] |
| Linalool | 74,6 | 3398,4 | 46 |
| 2-Methylbutanol | 15,9 | 235,1 | 15 |
| 2E-Hexenal | 10,7 | 282,5 | 26 |

(fortgesetzt)

| Name MI | "wässriges Aroma" [ppm] | "Aromakonzentrat" [ppm] | Anreicherung [-fach] |
|---|---|---|---|
| Octanal | 7,0 | 214,5 | 31 |
| Butanol | 4,6 | 55,4 | 12 |
| Geranial | 2,4 | 105,3 | 44 |
| Neral | 2,0 | 104,6 | 52 |
| Diacetyl | 0,5 | 0,0 | 0,0 |

Tabelle 3 ist zu entnehmen, dass außer Diacetyl alle Geschmacks- und/oder Duftstoffe angereichert werden. Die Geschmacks- und/oder Duftstoffe aus Gruppe ii), die einen negativen sensorischen Eindruck vermitteln, nämlich Butylalkohol (wird ca. 12fach angereichert) und 2-Methylbutanol (wird ca. 15fach angereichert), werden um den Faktor 1.7 weniger stark angereichert als die Geschmacks- und/oder Duftstoffe aus der Gruppe i), die einen positiven sensorischen Eindruck vermitteln, nämlich 2-E-Hexenal (wird ca. 26fach angereichert), Octanal (wird ca. 31fach angereichert), Geranial (wird ca. 44fach angereichert), Linalool (wird ca. 46fach angereichert) und Neral (wird ca. 52fach angereichert).

Sofern in der vorliegenden Erfindung inklusive der bevorzugten Ausgestaltungen von ein oder mehreren Geschmacks- und/oder Duftstoffen aus der Gruppe i) gesprochen wird, dann sind üblicherweise ein, zwei, drei, vier, fünf, sechs, sieben, acht, neun, zehn oder mehrere, bevorzugt, zwei oder mehrere, weiter bevorzugt fünf oder mehrere Geschmacks- und/oder Duftstoffe offenbart.

Gemäß der vorliegenden Erfindung stellen die Geschmacks- und/oder Duftstoffe aus der Gruppe i) solche Geschmacks- und/oder Duftstoffe dar, die positive sensorische Eindrücke für insbesondere folgende Produkte, aber auch andere Produkte, welche diese Inhaltstoffe umfassen, vermitteln:

Erdbeere: Ethylbutyrat, Ethylmethylbutyrat-2, Methylcapronat, Linalool, gamma-Decalacton,

Himbeere: alpha- und beta-Ionon, delta-Decalacton, Hydroxybenzylaceton, 3Z-Hexenol,

Apfel: 2E-Hexenol, 3Z-Hexenol, 2E-Hexenal, Hexanal, Ethylbutyrat, Ethylmethylbutyrat-2, beta-Damascenon,

Orange: Ethylbutyrat, Methylbutyrat, Octanal, Hexanal, Linalool,

Grapefruit: Nootkaton, Ethylbutyrat, Linalool, p-Menthenthiol-1,8,

Zitrone: Citral, Geraniol, Caryophyllen,

Kirsche: Benzaldehyd, 2E-Hexenol, 2E-Hexenal, Hexanal, beta-Damascenon,

Pfirsich: gamma-Decalacton, 2E-Hexenol, beta-Damascenon, Linalooloxid,

Banane: Isoamylacetat,

Birne: Ethyl-2E,4Z-decadienoat,

Schwarze Johannisbeere: 8-Mercapto-p-Menth-1-en-3-on,

Kaffee: beta-Damascenon, Furfurylthiol-2, 4-Vinylguaiacol, isomere Isopropylmethoxypyrazine, isomere Ethyldimethylpyrazine,

Grüntee: 3-Z-Hexenol, Indol, Methyljasmonat, Jasminlacton,

Zwiebel: Dipropyldisulfid, Dipropyltrisulfid, Methylpropyldisulfid,

Fleisch: 2E,4Z,7Z-Tridecatrienal, 2E,5Z-Undecadienal, 2E,4Z-Decadienal,

Reis: 2-Acetyl-1-pyrrolin; Octanal, Nonanal,

Milch: 1-Octen-3-on, 1-Octen-3-ol, 4-Ocanolid, 5-Octanolid,

Tomate: 3Z-Hexenol, beta-Damascenon,

Mint: L-Menthol, Menthon, L-Carvon,

Bier: Isoamylacetat, Phenylethanol, Ethylbutyrat und

Wein: Weinlacton, Menthofurolactone.

In einer weiteren bevorzugten Ausgestaltung werden die ein oder mehreren Geschmacks- und/oder Duftstoffe aus einer der Gruppen Erdbeere, Himbeere, Apfel, Orange, Grapefruit, Kirsche, Pfirsich, Kaffee, Grüntee und Zwiebel, Fleisch, Reis, Milch, Molke, Tomate, Mint, Bier und Wein wie vorstehend definiert, ausgewählt.

Im Rahmen der vorliegenden Erfindung bedeuten ein oder mehrere Geschmacks- und/oder Duftstoffe aus der Gruppe ii), die einen negativen sensorischen Eindruck vermitteln, dass ein, zwei, drei, vier, fünf, sechs, sieben, acht, neun, zehn oder mehrere, bevorzugt zwei oder mehrere, weiter bevorzugt drei oder mehrere, weiter bevorzugt fünf oder mehrere solcher Geschmack- und/oder Duftstoffe offenbart sind.

Gemäß einer weiteren bevorzugten Ausgestaltung der vorliegenden Erfindung umfassen die ein oder mehreren Geschmacks- und/oder Duftstoffe aus der Gruppe ii) ein oder mehrere Verbindungen ausgewählt aus der Gruppe bestehend aus niedermolekularen $C_1$-$C_5$-Alkoholen, bevorzugt Methanol, Ethanol, 1-Butanol, 2-Methylbutanol, 3-Methylbutanol; Diacetyl, Acetaldehyd, Milchsäure und Essigsäure, wenn ein oder mehrere Geschmacks- und/oder Duftstoffe aus der Gruppe i) ausgewählt werden aus Ethylbutyrat, Methylbutyrat, Octanal, Hexanal, Linalool, Nootkaton, p-Menthenthiol-1,8, Benzaldehyd, 2E-Hexenol, 2E-Hexenal, Hexanal, beta-Damascenon, gamma-Decalacton und Linalooloxid.

Gemäß einer weiteren bevorzugten Ausgestaltung der vorliegenden Erfindung umfassen die ein oder mehreren Geschmacks- und/oder Duftstoffe aus der Gruppe ii) Furfural und/oder Furfurylalkohol, wenn ein oder mehrere Geschmacks- und/oder Duftstoffe aus der Gruppe i) ausgewählt werden aus beta-Damascenon, Furfurylthiol, 4-Vinylguaiacol, Isopropylmethoxypyrazin und Ethyldimethylpyrazin.

Gemäß einer weiteren bevorzugten Ausgestaltung der vorliegenden Erfindung umfassen die ein oder mehreren Geschmacks- und/oder Duftstoffe aus der Gruppe ii) Phenol, wenn ein oder mehrere Geschmacks- und/oder Duftstoffe aus der Gruppe i) ausgewählt werden aus 3Z-Hexenol, Indol, Methyljasmonat und Jasminlacton.

Gemäß einer weiteren bevorzugten Ausgestaltung der vorliegenden Erfindung umfassen die ein oder mehreren Geschmacks- und/oder Duftstoffe aus der Gruppe ii) Methylmercaptan, wenn ein oder mehrere Geschmacks- und/oder Duftstoffe aus der Gruppe i) ausgewählt werden aus Dipropyldisulfid, Dipropylsulfid und Methylpropyldisulfid.

Erfindungsgemäß wird in Schritt c) des erfindungsgemäßen Adsorptionsverfahrens der Lösungsmittelanteil für ein Lösungsmittel, das kein Wasser ist, so eingestellt, dass der log Pow*-Wert eines oder mehrere Geschmacks- und/oder Duftstoffe aus der Gruppe i) inklusive der vorstehend beschriebenen bevorzugten Ausgestaltungen unabhängig voneinander jeweils einen Wert von 1,20 oder mehr, bevorzugt 1,50 oder mehr, weiter bevorzugt 1,70 oder mehr und ganz besonders bevorzugt 1,80 oder mehr aufweist. Durch die Einstellung des Lösungsmittelanteils in Schritt c) des erfindungsgemäßen Adsorptionsverfahrens auf diesen log Pow*-Wert erreicht man eine erfindungsgemäße Anreicherung der Geschmacks- und/oder Duftstoffe aus der Gruppe i) und gleichzeitig eine um den Faktor 1,5 oder mehr geringere Anreicherung ein oder mehreren Geschmacks- und/oder Duftstoffe aus der Gruppe ii).

Gemäß einer bevorzugten Ausgestaltung des erfindungsgemäßen Adsorptionsverfahrens wird in Schritt c) der Lösungsmittelanteil gleichzeitig so eingestellt wird, dass die ein oder mehreren Geschmacks- und/oder Duftstoffe aus der Gruppe ii), die einen negativen sensorischen Eindruck vermitteln, unabhängig voneinander, jeweils einen log Pow*-Wert von kleiner 1,20 aufweisen. Um das Verhältnis von ein oder mehreren Geschmack- und/oder Duftstoffen aus der Gruppe i) zu den ein oder mehreren Geschmack- und/oder Duftstoffen aus der Gruppe ii) im resultierenden Aromakonzentrat zugunsten der Geschmack- und/oder Duftstoffe aus der Gruppe i) zu verschieben, sollte der log Pow*-Wert des oder der Geschmack- und/oder Duftstoffe aus der Gruppe ii) vorzugsweise bei 1,1 oder weniger, weiter bevorzugt bei 1,0 oder weniger liegen.

In einer weiteren bevorzugten Ausgestaltung ist ein erfindungsgemäßes Adsorptionsverfahren dann besonders bevorzugt, wenn der Lösungsmittelanteil in Schritt c) im Bereich zwischen 1 Vol.-% und 6 Vol.-%, weiter bevorzugt im Bereich zwischen 2 Vol.-% und 5 Vol.-%, weiter bevorzugt im Bereich zwischen 3 Vol.-% und 4 Vol.-% jeweils bezogen auf die resultierende Lösung liegt.

Gemäß der vorliegenden Erfindung können in Schritt d) alle geeigneten Adsorptionsmaterialien verwendet werden, die üblicherweise für ein Adsorptions-/Desorptionsverfahren zur Verfügung stehen.

Die geeignete erfindungsgemäße Vorrichtung ist üblicherweise eine Säulen aus Glas oder Edelstahl, wobei das innere Volumen üblicherweise im Bereich von wenigen Millilitern bis zu tausend Litern, bevorzugt im Bereich von 1 L bis 500 L, weiter bevorzugt von 20 L bis 400 L liegt.

Bevorzugte Adsorptionsmaterialien, die in einer erfindungsgemäßen Säule verwendet werden, sind verschiedenartig vernetzte Polystyrole, bevorzugt Copolymerate aus Ethylvinylbenzol und Divinylbenzol, Vinylpyrrolidon und Divinylbenzol, Vinylpyridin und Divinylbenzol, Styrol und Divinylbenzol, aber auch andere Polymere, wie bevorzugt Polyaromate, Polystyrole, Poly(meth)acrylate, Polypropylene, Polyester, Polytetrafluorethylen etc.

Für das erfindungsgemäße Adsorptionsverfahren werden das oder die Lösungsmittel aus Schritt b) bevorzugt ausgewählt aus der Gruppe bestehend aus Methanol, Ethanol, Propanol und Isopropanol.

Weiterhin bevorzugt werden gemäß Schritt f) des erfindungsgemäßen Adsorptionsverfahrens das oder die Lösungsmittel ausgewählt aus der Gruppe bestehend aus Methanol, Ethanol, Propanol, Isopropanol, Ethylacetat, Diacetin, Triacetin, flüssiges Kohlendioxid, lebensmittelgeeignete Fluorchlorkohlenwasserstoffe und Pflanzentriglyceride. Bevorzugt werden Ethanol, Propanol, Isopropanol, Ethylacetat, Diacetin und Triacetin verwendet. Weiter bevorzugt werden Ethanol und Isopropanol und insbesondere bevorzugt wird Ethanol als Lösungsmittel in Schritt f) des erfindungsgemäßen Adsorptionsverfahrens verwendet. Ethanol ist insbesondere deshalb am meisten bevorzugt, da das resultierende Aromakonzentrat direkt zur Herstellung der erfindungsgemäßen Produkte ohne vorherige Abdestillation und damit Temperatureinwirkung verwendet werden kann.

In einer weiteren bevorzugten Ausgestaltung des erfindungsgemäßen Adsorptionsverfahrens wird die Strömungsgeschwindigkeit der Lösung aus Schritt e) zumindest teilweise während des Adsorptionsvorganges in Schritt c) im Bereich von 0,1 bis 2,5 cm/sec eingestellt. Der Parameter der Strömungsgeschwindigkeit ist in diesem Zusammenhang für die Ausbildung der lokalen Verteilungskoeffizienten der ein oder mehreren Geschmack- und/oder Duftstoffe zwischen dem Adsorptionsmaterial und dem wässrigen Aroma mit verantwortlich. Bevorzugt liegt die Strömungsgeschwindigkeit im Bereich von 0,2 bis 1,5 cm/sec, weiter bevorzugt im Bereich von 0,4 bis 0,9 cm/sec.

In einer weiteren bevorzugten Ausgestaltung des vorliegenden erfindungsgemäßen Adsorptionsverfahren liegt die Temperatur der Lösung aus Schritt c) zumindest teilweise während des Adsorptionsvorganges in Schritt e) des erfindungsgemäßen Adsorptionsverfahrens im Bereich von 0° C bis 80° C. Der Parameter der Temperatur ist ebenfalls mitverantwortlich für die Ausbildung des lokalen Verteilungskoeffizienten. Weiterhin bevorzugt ist ein Temperaturbereich von 10 bis 50°C, besonders bevorzugt ein Temperaturbereich von 20 bis 40°C.

In einer weiteren bevorzugten Ausgestaltung liegt der Gegendruck innerhalb der Vorrichtung aus Schritt d) während des Adsorptionsvorgangs in Schritt e) im Bereich von 0,1 bar bis 4,0 bar. Der Gegendruck innerhalb der Vorrichtung aus Schritt d) ist derjenige Druck, der durch den Widerstand des Adsorptionsmaterials entsteht, wenn man die Lösung in Schritt c) des erfindungsgemäßen Adsorptionsverfahrens durch die Vorrichtung aus Schritt d) pumpt. Bevorzugt ist ein Gegendruck im Bereich von 0,3 bar bis 2,5 bar, besonders bevorzugt von 0,8 bar bis 1,5 bar.

In einer weiteren bevorzugten Ausgestaltung des vorliegenden erfindungsgemäßen Adsorptions- und/oder Desorptionsverfahrens wird das wässrige Aroma unter Verwendung eines (vorzugsweise komprimierten) Gases in einer erfindungsgemäß einzusetzenden Vorrichtung transportiert. Bevorzugte Gase sind inerte Gase, insbesondere die Inertgase Argon, Stickstoff und Kohlendioxid. Die Verwendung von Inertgasen zur Unterstützung des Transportes der wässrigen Aromen beim erfindungsgemäßen Adsorptions- und/oder Desorptionsverfahren ist besonders bei oxidationsanfälligen, d.h. leicht oxidierbaren, Aromen oder Aromastoffen von Vorteil. Oxidationsanfällige Aromen oder Aromastoffe können bei Durchführung des erfindungsgemäßen Adsorptions- und/oder Desorptionsverfahrens ohne Verwendung von Inertgas eine Veränderung des sensorischen Profils erleiden, was zur Bildung von unerwünschten Noten (Fehlnoten) führen kann. Besonders oxidationsanfällig sind schwefelhaltige Aromastoffe und bestimmte, dem Fachmann bekannte Substanzklassen wie beispielsweise Aldehyde. Typische schwefelhaltige Aromastoffe sind hier Dipropylsulfid, Dipropenylsulfid, Diallylsulfid, Methypropenylsulfid, sowie entsprechende Di-, Tri- und Tetrasulfide der genannten Sulfide. Insbesondere sind die Aromen / Aromastoffe von Kaffee, Zwiebeln und anderen Allium-Gewächsen, sowie Zitrusfrüchten, wie beispielsweise Orange, Zitrone oder Grapefruit, zu nennen, wobei besonders die schwefelhaltigen Aromastoffe, aber auch Aldehyde durch das bevorzugte erfindungsgemäße Verfahren vor Oxidation geschützt werden können. Besonders relevante Aromen / Aromastoffe sind hier insbesondere Furfurylmethylsulfid und dessen Disulfid (insbesondere im Bereich der Zwiebelaromen) sowie Furfurylthiol-2 (insbesondere im Bereich der Kaffeearomen). Wichtige aromarelevante Aldehyde sind insbesondere n-Hexanal, 2E- bzw 3Z-Hexenal, n-Octanal, n-Decanal, n-Dodecanal und Citral, die wichtige Aromabestandteile, insbesondere von Zitrusfrüchten, darstellen.

Bevorzugt sind somit auch ein erfindungsgemäßes Verfahren, bei dem beim Adsorptionsverfahren und/oder Desorptionsverfahren (siehe unten) ein Inertgas zum Transport der flüssigen Medien verwendet wird, insbesondere zum Schutz der genannten oxidationsanfälligen Aromen bzw. Aromastoffen.

Im Gegensatz zum erfindungsgemäßen Adsorptionsverfahren werden bei dem erfindungsgemäßen Desorptionsverfahren keine gezielten Diskriminierungen von Geschmack- und/oder Duftstoffen bei der Adsorption an das Adsorptionsmaterial im Schritt c) vorgenommen, sondem die Diskriminierung der ein oder mehreren Geschmacks- und/oder Duftstoffe aus der Gruppe ii) wird durch gezieltes Desorbieren in Schritt e) des erfindungsgemäßen Desorptionsverfahrens vorgenommen, wobei das zur Desorption verwendete oder die zur Desorption verwendeten Lösungsmittel so gewählt werden, dass die zu desorbierenden Geschmacks- und/oder Duftstoffe einen log Pow* Wert von 1,20 oder kleiner besitzen.

Im Weiteren werden bevorzugte Ausgestaltungen in Bezug auf das erfindungsgemäße Desorptionsverfahren dargestellt. Sofern vorstehend bereits Aussagen zu den eingesetzten erfindungsgemäßen Materialien getroffen wurden, beispielsweise den Geschmacks- und/oder Duftstoffen aus der Gruppe i) oder der Gruppe ii), des wässrigen Aromas, dem resultierenden Aromakonzentrat, dem Adsorptionsmaterial, der geeigneten Vorrichtung, den Lösungsmitteln, so sind diese auch auf das erfindungsgemäße Desorptionsverfahren anzuwenden.

Eine bevorzugte Ausgestaltung des erfindungsgemäßen Desorptionsverfahrens ist dadurch gekennzeichnet, dass die Strömungsgeschwindigkeit der Lösung aus Schritt d) zumindest teilweise während des Desorptionsvorgangs in Schritt e) im Bereich von 1 bis 5 cm/min liegt. Die Strömungsgeschwindigkeit, die im Zusammenhang mit dem erfindungsgemäßen Adsorptionsverfahren beschrieben wurde, findet auf das erfindungsgemäße Desorptionsverfahren keine Anwendung.

In einer weiteren bevorzugten Ausgestaltung liegt die Temperatur der Lösung aus Schritt d) des erfindungsgemäßen Desorptionsverfahrens zumindest teilweise während des Desorptionsvorgangs in Schritt e) im Bereich von 0° C bis 80° C. Der Parameter der Temperatur ist ebenfalls mitverantwortlich für die Ausbildung des lokalen Verteilungskoeffizienten. Weiterhin bevorzugt ist ein Temperaturbereich von 10 bis 50° C, besonders bevorzugt ein Temperaturbereich von 20 bis 40°C.

In einer weiteren bevorzugten Ausgestaltung ist das erfindungsgemäße Desorptionsverfahren dadurch gekennzeichnet, dass der Gegendruck innerhalb der Vorrichtung aus Schritt b) während des Desorptionsvorgangs im Schritt e) im Bereich von 0,05 bar bis 2,0 bar liegt. Der Gegendruck innerhalb der Vorrichtung aus Schritt b) ist derjenige Druck, der durch den Widerstand des Adsorptionsmaterials entsteht, wenn man die Lösung in Schritt e) des erfindungsgemäßen Desorptionsverfahrens durch die Vorrichtung aus Schritt b) pumpt und durch ein Manometer angezeigt wird. Bevorzugt ist ein Gegendruck im Bereich von 0,1 bar bis 1,5 bar, besonders bevorzugt von 0,2 bar bis 1,0 bar.

Gemäß einer weiteren bevorzugten Ausgestaltung der erfindungsgemäßen Adsorptions- und Desorptionsverfahren können die Richtungen des Adsorptionsvorgangs und des Desorptionsvorgangs, also die Schritte d) und g) der erfindungsgemäßen Adsorptionsverfahrens und die Schritte c) und des Desorptionsvorgangs im Schritt e) des erfindungsgemäßen Desorptionsverfahrens gleichgerichtet oder entgegengesetzt sein.

Gemäß der vorliegenden Erfindung ist ein zum Verzehr geeignetes Produkt bevorzugt ein Nahrungsmittel, Genussmittel und/oder Getränk, das dazu bestimmt ist, in eine Mundhöhle eingebracht zu werden, dort eine bestimmte Zeit zu verbleiben und anschließend entweder geschluckt, d. h. verzehrt (z. B. Lebensmittel) oder wieder aus der Mundhöhle entfernt zu werden (beispielsweise Kaugummis). Hierzu zählen auch alle Stoffe oder Erzeugnisse, die dazu bestimmt sind, in verarbeitetem, teilweise verarbeitetem oder unverarbeitetem Zustand von Menschen oder Tieren aufgenommen zu werden. Insofern zählen auch alle Stoffe dazu, die dem Lebensmittel bei seiner Herstellung, Verarbeitung oder Bearbeitung zugesetzt werden.

Kaugummis umfassen im Allgemeinen aus einer Kaugummibase, d. h. einer beim Kauen plastisch werdenden Kaufmasse, aus Zucker in verschiedenen Arten, Zuckeraustauschstoffen, Süßstoffen, Zuckeralkoholen, Feuchthaltemitteln, Verdickern, Emulgatoren, verkapselte und/oder nicht verkapselte Aromen und/oder Stabilisatoren.

Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstearat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerin-monostearat.

Bevorzugte, zum Verzehr geeignete aromatisierte Produkte (Nahrungsmittel, Genussmittel, Getränke), werden ausgewählt aus der Gruppe, bestehend aus Backwaren, vorzugsweise ausgewählt aus der Gruppe bestehend aus Brot, Trockenkekse, Kuchen und sonstiges Gebäck; Süßwaren, vorzugsweise ausgewählt aus der Gruppe bestehend aus Schokolade, Schokoladenriegelprodukte, sonstige Riegelprodukte, Fruchtgummi, Hart- und Weichkaramellen und Kaugummi; alkoholische oder nicht-alkoholische Getränke, vorzugsweise ausgewählt aus der Gruppe bestehend aus Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte und Frucht- oder Gemüsesaftzubereitungen; Instantgetränke, vorzugsweise ausgewählt aus der Gruppe bestehend aus Instant-Kakao-Getränke, Instant-Tee-Getränke und Instant-Kaffeegetränke; Fleischprodukte, vorzugsweise ausgewählt aus der Gruppe bestehend aus Schinken, Frischwurst- oder Rohwurstzubereitungen und gewürzte oder marinierte Frisch- oder Pökelfleischprodukte; Eier oder Eiprodukte, vorzugsweise ausgewählt aus der Gruppe bestehend aus Trockenei, Eiweiß und Eigelb; Getreideprodukte, vorzugsweise ausgewählt aus der Gruppe bestehend aus Frühstückscerealien, Müsliriegel und vorgegarte Fertigreis-Produkte; Milchprodukte, vorzugsweise ausgewählt aus der Gruppe bestehend aus Milchgetränke, Milcheis, Joghurt, Kefir, Frischkäse, Weichkäse, Hartkäse, Trockenmilchpulver, Molke, Butter, Buttermilch und teilweise oder ganz hydrolisierte Milchprotein-haltige Produkte; Produkte aus Sojaprotein oder anderen Sojabohnen-Fraktionen, vorzugsweise ausgewählt aus der Gruppe bestehend aus Sojamilch und daraus gefertigte Produkte, Sojalecithin-haltige Zubereitungen, fermentierte Produkte wie Tofu oder Tempe oder daraus gefertigte Produkte und Sojasoßen; Fruchtzubereitungen, vorzugsweise ausgewählt aus der Gruppe bestehend aus Konfitüren, Fruchteis, Fruchtsoßen und Fruchtfüllungen; Gemüsezubereitungen, vorzugsweise ausgewählt aus der Gruppe bestehend aus Ketchup, Soßen, Trockengemüse, Tiefkühlgemüse, vorgegarte Gemüse, in Essig eingelegte Gemüse und eingekochte Gemüse; Knabberartikel, vorzugsweise ausgewählt aus der Gruppe bestehend aus gebackenen oder frittierten Kartoffelchips oder Kartoffelteigprodukte, Brotteigprodukte und Extrudate auf Mais- oder Erdnussbasis; Produkte auf Fett- und Ölbasis oder Emulsionen derselben, vorzugsweise ausgewählt aus der Gruppe bestehend aus Mayonnaise, Remoulade, Dressings und Würzzubereitungen; sonstige Fertiggerichte und Suppen, vorzugsweise ausgewählt aus der Gruppe bestehend aus Trockensuppen, Instant-Suppen, vorgegarte Suppen, Gewürze, Würzmischungen und Aufstreuwürzungen (englisch: Seasonings), die beispielsweise im Snackbereich Anwendung finden. Der Gesamtanteil an erfindungsgemäßen Aromakonzentraten sowie deren bevorzugten Ausgestaltungen in einem erfindungsgemäßen (zu Verzehr geeigneten) Produkt liegt, je nach Produkttyp, regelmäßig im Bereich von 0,001 bis 10000 ppm, bevorzugt im Bereich von 0,01 bis 100 ppm in der Endanwendung.

Die zum Verzehr geeigneten Produkte im Sinne der vorliegenden Erfindung können auch als Halbfertigwaren zur Herstellung weiterer erfindungsgemäßer (zum Verzehr geeigneter) Produkte, vorzugsweise Nahrungsmittel- Genussmittel und/oder Getränke Produkte, eingesetzt werden. Hierbei werden die erfindungsgemäßen Halbfertigwaren zur Aromatisierung von daraus gefertigten Produkten als Fertigwaren verwendet werden. Der Gesamtanteil an erfindungsgemäßen Aromakonzentraten in einem erfindungsgemäßen Halbfertigprodukt liegt, je nach Produkttyp, regelmäßig im Bereich von 0,01 bis 10000 ppm, bevorzugt im Bereich von 0,02 bis 200 ppm.

Erfindungsgemäße kosmetische oder pharmazeutische Zubereitungen lassen sich auch als Emulsion vom Typ " Wasser-in-Öl" (W/O), vom Typ "Öl-in-Wasser" (O/W), oder multiple Emulsionen, z.B. vom Typ Wasser-in-Öl-in-Wasser (W/O/W), PIT Emulsion, Pickering-Emulsion, Mikro-Emulsion oder Nano-Emulsion formulieren; besonders bevorzugte Emulsionen sind vom Typ "Öl-in-Wasser" (O/W), oder vom Typ Wasser-in-Öl-in-Wasser (W/O/W). Die erfindungsgemäßen kosmetischen Zubereitungen können ferner insbesondere formuliert sein als, Stift, Stick, Aerosol, Spray, sprühfähigen Emulsionen, Schaum, Tränkungslösung, z.B. für kosmetische Tücher (englisch: wipes), Reinigungsmittel wie Reinigungsmilch, Reinigungslotionen auf wässriger, alkoholischer oder glycolischer Basis, Seife, Syndets, Hautpflegemittel, Creme, Lotion, Milch, Emulsionsschaum, Mikro-, oder Nanoemulsion, Paste, Gel (z.B. Hydro- oder Hydrodispersionsgel), Balsam, Serum, Roll-On, Pumpspray, Aerosol (schäumend, nicht schäumend oder nach-schäumend), Hautpflegemittel, Fußpflegemittel (inklusive Keratolytika, Desodorantien), Insekten abwehrendes Mittel, Sonnenschutzmittel, Aftersun-Präparat, Rasiermittel, Haarentfernungsmittel, Haarpflegemittel wie z.B. Shampoo, 2-in-1 Shampoo, Anti-Schuppen-Shampoo, Babyshampoo, Shampoo für trockene Kopfhaut, Shampookonzentrat, Conditioner, Haarkur, Haarwasser, Haarspülung, Frisiercrème, Dauerwell- und Fixierungsmittel, Haarglättungsmittel (Entkräuselungsmittel, Relaxer), Haarfestiger (Spray), Styling-Aid (z.B. Gel), als Blondiermittel, Haaraufheller, Haarconditioner, Haarmousse, Haartönung, Deodorant und / oder Antitranspirant; Mundwasser und Munddusche, Aftershave Balm, Pre- und Aftershave Lotion, Augenpflege, Make-Up, Make-Up Entferner, Babyartikel, Badeartikel (z.B. Kapsel), oder Maske.

Unter Mundhygieneprodukten werden in der vorliegenden Erfindung die dem Fachmann geläufigen Formulierungen zur Reinigung und Pflege der Mundhöhle und des Rachenraumes sowie zur Erfrischung des Atems verstanden. Bekannte und gebräuchliche mundhygienische Formulierungen sind sowohl Cremes, Gele, Pasten, Schäume, Emulsionen, Suspensionen, Areosole, Sprays als auch Kapseln, Granulate, Pastillen, Tabletten, Bonbons oder Kaugummis, ohne dass diese Aufzählung an Darreichungsformen bzgl. der Einsatzmöglichkeiten limitierend ist. Solche Formulierungen dienen dazu, Zahnsubstanz und Mundhöhle zu reinigen und zu pflegen sowie den Atem zu erfrischen.

Erfindungsgemäße Mundhygieneprodukte werden bevorzugt ausgewählt aus der Gruppe bestehend aus: Zahncremes, Zahnpasten, Zahngele, Mundwässer, Mundspülungen, Flüssigkeiten zum Gurgeln, Mund- oder Rachensprays (Pump- oder Aerosolspray), Lutschpastillen, Lutschtabletten, Bonbons, Kaugummis, Kaubonbons und zahnpflegende Kaugummis.

Weiter bevorzugt werden Mundhygieneprodukte ausgewählt aus der Gruppe bestehend aus Zahncremes, Zahnpasten, Zahngele, Mund- oder Rachensprays (Pump- oder Aerosolspray), Lutschpastillen, Lutschtabletten, Bonbons, Kaugummis, Kaubonbons und zahnpflegende Kaugummis.

Zahnpflegemittel (als Beispiel für der Mundpflege dienende erfindungsgemäße Zubereitungen) umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite®, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Mentholderivate (z.B. L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Der Gesamtanteil an erfindungsgemäßen Aromakonzentraten in einem erfindungsgemäßen Mundhygieneprodukt liegt, je nach Produkttyp, regelmäßig im Bereich von 0,001 bis 10000 ppm, bevorzugt im Bereich von 0,1 bis 1000 ppm.

Produkte im Sinne der vorliegenden Erfindung, bevorzugt pharmazeutische Produkte, können auch in Form von Kapseln, Tabletten (nicht-überzogene sowie überzogene Tabletten, beispielsweise magensaftresistente Überzüge), Dragees, Granulate, Pellets, Feststoffmischungen, Dispersionen in flüssigen Phasen, als Emulsionen, als Pulver, als Lösungen, als Pasten oder als andere schluck- oder kaubare Zubereitungen oder als Nahrungsergänzungsmittel vorliegen.

Als weitere Bestandteile für die erfindungsgemäßen Produkte, insbesondere Nahrungsmittel-, Genussmittel-, Getränke-, Halbfertig-, Mundhygiene-, kosmetische oder pharmazeutische Produkte, werden übliche Grund-, Hilfs- und/oder Zusatzstoffe verwendet. Vorzugsweise werden diese weiteren Bestandteile ausgewählt aus der Gruppe, bestehend aus Wasser, Gemische frischer oder prozessierter, pflanzlicher oder tierischer Grund- oder Rohstoffe, verdauliche oder nicht-verdauliche Kohlenhydrate (beispielsweise Saccharose, Maltose, Fruktose, Glukose, Dextrine, Amylose, Amylopektin, Inulin, Xylane, Cellulose), Zuckeralkohole (beispielsweise Sorbit, Mannitol, Xylitol), natürliche oder gehärtete Fette (beispielsweise Talg, Schmalz, Palmfett, Kokosfett, gehärtetes Pflanzenfett), fette Öle (beispielsweise Sonnenblumenöl, Erdnussöl, Maiskeimöl, Distelöl, Olivenöl, Walnussöl, Fischöl, Sojaöl, Sesamöl), Fettsäuren oder deren Salze (beispielsweise Kaliumstearat, Kaliumpalmitat), proteinogene oder nicht-proteinogene Aminosäuren und verwandte Verbindungen (beispielsweise Taurin, Creatin, Creatinin), Peptide, native oder prozessierte Proteine (beispielsweise Gelatine), Enzyme (beispielsweise Peptidasen, Glucosidasen, Lipasen, Proteinasen), Nukleinsäuren, Nukleotide (Inositolphosphat), geschmacksmodulierende Stoffe (beispielsweise Natriumglutamat, 2-Phenoxypropionsäure), Emulgatoren (beispielsweise Lecitine, Diacylglycerole), Stabilisatoren (beispielsweise Carrageenan, Alginat, Johannisbrotkernmehl, Guarkernmehl), Konservierungsstoffe (beispielsweise Benzoesäure, Sorbinsäure), Antioxidantien (beispielsweise Tocopherol, Ascorbinsäure), Gelatoren (beispielsweise Zitronensäure), organische oder anorganische Säuerungsmittel (Äpfelsäure, Essigsäure, Zitronensäure, Weinsäure, Phosphorsäure), Bitterstoffe (beispielsweise Chinin, Koffein, Limonin), Süßstoffe (beispielsweise Saccharin, Cyclamat, Aspartam, Neotam, Neohesperidindihydrochalcon), mineralische Salze (beispielsweise Natriumchlorid, Kaliumchlorid, Magnesiumchlorid, Natriumphosphate), die enzymatische Bräunung verhindernde Stoffe (beispielsweise Sulfit, Ascorbinsäure), ätherische Öle, Pflanzenextrakte, natürliche oder synthetische Farbstoffe oder Farbpigmente (beispielsweise Carotinoide, Flavonoide, Antocyane, Chlorophyll und deren Derivate, Gewürze sowie Riechstoffe, synthetische, natürliche oder naturidentische Geschmacks- und/oder Duftstoffe.

Die erfindungsgemäßen Produkte, insbesondere Nahrungsmittel-, Genussmittel-, Getränke-, Halbfertig-, Mundhygiene-, kosmetische oder pharmazeutische Produkte, bevorzugt Mundhygieneprodukte umfassen bevorzugt ein oder mehrere Grund-, Hilfs- und/oder Zusatzstoffe aus folgender Gruppe:

Konservierungsmittel, Abrasiva (schleifende Mittel), weitere antibakterielle Mittel, entzündungshemmende Mittel, irritationsverhindernde Mittel, irritationshemmende Mittel, weitere antimikrobielle Mittel, Antioxidantien, Adstringentien, Antistatika, Binder, (mineralische) Füllstoffe, Puffer, Trägermaterialien, Chelatoren (Chelatbildner), reinigende Mittel, pflegende Mittel, oberflächenaktive Substanzen, deodorierende Mittel, Emulgatoren, Enzyme, Fasern, Filmbildner (filmbildende Substanzen), Fixateure, Schaumbildner, Substanzen zum Verhindern des Schäumens, Schaumstabilisatoren, Schaumbooster, gelierende Mittel, gelbildende Mittel, feuchtigkeitsspendende Mittel (Moisturizer), anfeuchtende Substanzen, feuchthaltende Substanzen, bleichende Mittel, aufhellende Mittel (z.B. Wasserstoffperoxid), imprägnierende Mittel, reibungsverringernde Mittel, Gleitmittel, geruchs- und/oder geschmacksmodulierende Mittel, geruchs- und/oder geschmacksreduzierende Mittel, geruchs- und/oder geschmacksverstärkende Mittel, Trübungsmittel, plastifizierende Mittel, deckfähige Mittel, Glanzmittel, Silikone, (schleim)hautkühlende Mittel (Kühlwirkstoffe), (schleim)hautberuhigende Mittel, (schleim)hautreinigende Mittel, (schleim)hautpflegende Mittel, (schleim)hautheilende Mittel, schleimhautschützende Mittel, UV-Filter, Stabilisatoren, suspendierende Mittel, Vitamine, fette Öle, Wachse, Fette, Phospholipide, gesättigte Fettsäuren, ein- oder mehrfach ungesättigte Fettsäuren, alpha-Hydroxysäuren, Polyhydroxysäuren, Verflüssiger, Farbstoffe, farbschützende Mittel, Pigmente, Tenside, Elektrolyte, Silikonderivate, Polyole, organische Lösungsmittel, Kieselsäuren, Calciumcarbonat, Calciumhydrogenphosphat, Aluminiumoxid, Fluoride, Zink-, Zinn-, Kalium-, Natrium- und Strontiumsalze, Pyrophosphate, Hydroxyapatite.

Sofern das erfindungsgemäße Produktzubereitung eine Lösung oder Lotion darstellt, können beispielsweise als Lösungsmittel verwendet werden: Wasser oder wässrige Lösungen, Öle, wie Triglyceride der Caprin- oder der Caprylsäure oder auch Alkohole, Diole oder Polyole niedriger C-Zahl sowie deren Ether, vorzugsweise Ethanol, Isopropanol, Propylenglykol, Glycerin, Ethylenglykol. Insbesondere werden Gemische der vorstehend genannten Lösungsmittel verwendet.

Erfindungsgemäße Produktzubereitungen, bevorzugt solche, die für den Einsatz als Zahn- und/oder Mundpflegeprodukt vorgesehen sind, sind bevorzugt frei von kariogenen Substanzen, insbesondere frei von Saccharose, Glucose, Lactose, hydrolisierter Lactose, Sorbose, Arabinose, Xylose, Mannose, Maltose, Galactose, Maltotriose und Fructose.

In einer weiteren bevorzugten Ausgestaltung umfassen die erfindungsgemäßen Produkte, insbesondere Nahrungsmittel-, Genussmittel-, Getränke-, Halbfertig-, Mundhygiene-, kosmetische oder pharmazeutische Produkte, weitere Geschmacks- und/oder Duftstoffe enthalten, die nicht in der Gruppe i) oder ii), wie vorstehend dargestellt, aufgeführt sind.

Die weiteren Geschmacks- und/oder Duftstoffe im Sinne der vorliegenden Erfindung umfassen somit unter anderem (schleimhaut)kühlende Mittel, (schleimhaut)wärmende Mittel, scharf schmeckene Stoffe, Süßstoffe, Zuckeraustauschstoffe, organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), und verzehrbare mineralische Salze (z.B. Natriumchlorid, Kaliumchlorid, Magnesiumchlorid, Natriumphosphate).

Geeignete Zuckeraustauschstoffe, die Bestandteil der erfindungsgemäßen Zubereitungen sein können, sind Zuckeralkohole wie beispielsweise Mannit, Sorbit und Sorbitsirup, Isomalt (z.B. Palatinit®), Maltit und Maltitsirup, Lactit, Xylit, Erythrit, Leucrose, Arabinol, Arabitol, Adonitol, Alditol, Ducitol, Iditol, aber auch Fructooligosaccharide (z.B. Raftilose®), Oligofructose oder Polydextrose.

Als typische Süßstoffe, die Bestandteil der erfindungsgemäßen Produkte sein können, seien Saccharin (gegebenenfalls als Na-, K- oder Ca-Salz), Aspartam (z.B. NutraSweet®) Cyclamat (gegebenenfalls als Na- oder Ca-Salz), Acesulfam-K (z.B. Sunett®), Thaumatin oder Neohesperidin-Dihydrochalkon genannt. Ferner können auch andere Süßstoffe wie Steviosid, Rebaudiosid A, Glycyrrhizin, Ultrasüß, Osladin, Brazzein, Miraculin, Pentadin, Phyllodulcin, Dihydrochalcone, Arylharnstoffe, trisubstituierte Guanidine, Glycyrrhizin, Superaspartam, Suosan, Sucralose (Trichlorgalactosaccarose, TGS), Alitam, Monellin oder Neotame® verwendet werden.

Bevorzugte scharf schmeckende und/oder den Speichelfluss im Mund anregende Substanzen und/oder ein Gefühl von Wärme und/oder ein kribbelndes Gefühl auf der Haut oder auf den Schleimhäuten hervorrufende Substanzen, die Bestandteil der erfindungsgemäßen Produkte sein können, sind z.B.: Capsaicin, Dihydrocapsaicin, Gingerole, Paradole, Shogaole, Piperin, Carbonsäure-N-vanillylamide, insbesondere Nonansäure-N-vanillylamid, Pellitorin oder Spilanthol, 2-Nonensäureamide, insbesondere 2-Nonensäure-N-isobutylamid, 2-Nonensäure-N-4-hydroxy-3-methoxyphenylamid, Alkylether von 4-Hydroxy-3-methoxybenzylalkohol, insbesondere 4-Hydroxy-3-methoxybenzyl-n-butylether, Alkylether von 4-Acyloxy-3-methoxybenzyl-alkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesondere (4-Hydroxy-3-methoxyphenyl)essigsäure-N-n-octylamid, Vanillomandelsäurealkylamide, Ferulasäure-phenethylamide, Nicotinaldehyd, Methylnicotinat, Propylnicotinat, 2-Butoxyethylnicotinat, Benzylnicotinat, 1-Acetoxychavicol, Polygodial und Isodrimeninol, weiter bevorzugt cis- und/oder trans-Pellitorin gemäß WO 2004/000787 bzw. WO 2004/043906, Alkencarbonsäure-N-alkylamide gemäß WO 2005/044778, Mandelsäurealkylamide gemäß WO 03/106404 oder Alkyloxyalkansäureamide gemäß WO 2006/003210.

Bevorzugte scharf schmeckende und/oder ein Gefühl von Wärme und/oder eine kribbelndes Gefühl auf der Haut oder auf den Schleimhäuten hervorrufende natürliche Extrakte, die Bestandteil der erfindungsgemäßen Produkte sein können, sind z.B.: Extrakte aus Paprika, Extrakte aus Pfeffer (z.B. Capsicum Extrakt), Extrakte aus Chili-Pfeffer, Extrakte aus Ingwerwurzeln, Extrakte aus Aframomum melgueta, Extrakte aus Spilanthes-acmella, Extrakte aus Kaempferia galanga oder Extrakte aus Alpinia galanga.

Bevorzugte Stoffe zur Maskierung eines oder mehrerer unangenehmer Geschmackseindrücke, insbesondere eines bitteren, adstringierenden und/oder metallischen Geschmackseindrucks oder Nachgeschmacks, die Bestandteil der erfindungsgemäßen Produkte sein können, sind: Lactisol [20-(4-Methoxyphenyl)milchsäure] (vgl. US 5,045,336), 2,4-Dihydroxybenzoesäure-Kaliumsalz (vgl. US 5,643,941), Ingwerextrakten (vgl. GB 2,380,936), Neohesperidindihydrochalcon (vgl. Manufacturing Chemist 2000, Juli-Heft, S. 16-17), bestimmte Flavone (2-Phenylchrom-2-en-4-one) (vgl. US 5,580,545), bestimmte Nucleotide, wie beispielsweise Cytidin-5'-monophosphate (CMP) (vgl. US 2002/0177576), bestimmte Natriumsalze wie Natriumchlorid, Natriumcitrat, Natriumacetat und Natriumlactat (vgl. Nature, 1997, Bd. 387, S. 563), ein Lipoprotein aus β-Lactoglobulin und Phosphatidinsäure (vgl. EP A 635 218), Neodiosmin [5,7-Dihydroxy-2-(4-methoxy-3-hydroxyphenyl)-7-O-neohesperidosyl-chrom-2-en-4-on] (vgl. US 4,154,862), vorzugsweise Hydroxyflavanone gemäß EP 1 258 200, dabei wiederum bevorzugt 2-(4-Hydroxyphenyl)-5,7-dihydroxychroman-4-on (Naringenin), 2-(3,4-Dihydroxyphenyl)-5,7-dihydroxychroman-4-on (Eriodictyol), 2-(3,4-Dihydroxyphenyl)-5-hydroxy-7-methoxychroman-4-on (Eriodictyol-7-methylether), 2-(3,4-Dihydroxyphenyl)-7-hydroxy-5-methoxychroman-4-on (Eriodictyol-5-methylether) und 2-(4-Hydroxy-3-methoxyphenyl)-5,7-dihydroxychroman-4-on (Homoeriodictyol), deren (2S)- oder (2R)-Enantiomere oder Gemische derselben sowie deren ein- oder mehrwertigen Phenolatsalze mit $Na^+$, $K^+$, $NH^{4+}$, $Ca^{2+}$, $Mg^{2+}$ oder $Al^{3+}$ als Gegenkationen, oder y-Aminobuttersäure (4-Aminobutansäure, als neutrale Form ("inneres Salz") oder in der Carboxylat- oder Ammoniumform) gemäß WO 2005/096841.

Stoffe, die bitter, adstringierend, pappig, staubig, trocken, mehlig, ranzig oder metallisch schmecken, sind beispielsweise: Xanthinalkaloide Xanthine (Coffein, Theobromin, Theophyllin), Alkaloide (Chinin, Brucin, Nicotin), phenolische Glycoside (z.B. Salicin, Arbutin), Flavonoidglycoside (z.B. Hesperidin, Naringin), Chalcone und Chalconglycoside, hydrolisierbare Tannine (Gallus- oder Elagsäureester von Kohlenhydraten, z.B. Pentagalloylglucose), nichthydrolysierbare Tannine (ggfs. galloylierte Catechine oder Epicatechine und deren Oligomere, z.B. Proanthyocyanidine oder Procyanidine, Thearubigenin), Flavone (z.B. Quercetin, Taxifolin, Myricetin), andere Polyphenole (g-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsulfat), bestimmte pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), bestimmte Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Kaliumchlorid, Magnesiumsalze, Eisensalze, Aluminiumsalze, Zinksalze, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin und Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Stoffe, die einen bitteren, adstringierenden, pappigen, staubigen, trockenen, mehligen, ranzigen oder metallischen Nachgeschmack haben, können beispielsweise zur Gruppe der Süßstoffe oder der Zuckeraustauschstoffe gehören. Beipielsweise seien genannt: Aspartam, Neotam, Superaspartam, Saccharin, Sucralose, Tagatose, Monellin, Stevioside, Thaumatin, Miraculin, Glycyrrhizin und deren Derivate, Cyclamat und die pharmazeutisch akzeptablen Salze der vorgenannten Verbindungen.

Vorteilhafte Zusatzstoffe zur Einarbeitung in die erfindungsgemäßen Produkte sind Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), Pflanzenextrakte, natürliche oder synthetische Farbstoffe oder Farbpigmente (z.B. Carotinoide, Flavonoide, Anthocyane, Chlorophyll und deren Derivate).

Erfindungsgemäße Produkte, insbesondere Nahrungsmittel-, Genussmittel-, Getränke-, Halbfertig-, Mundhygiene-, kosmetische oder pharmazeutische Produkte, können ferner Antioxidantien enthalten bzw. Stoffe, die eine antioxidative Wirkung verstärken können, vorzugsweise natürlich vorkommende Tocopherole und deren Derivate (z.B. Vitamin E - acetat), Vitamin C und ihre Salze bzw. Derivate (z.B. Ascorbylpalmitat, Mg - Ascorbylphosphat, Ascorbylacetat), Vitamin A und Derivate (Vitamin A - palmitat), Tocotrienole, Flavonoide, alpha-Hydroxysäuren (z.B. Citronensäure, Milchsäure, Apfelsäure, Weinsäure) und deren Na-, Ka- und Ca-Salze, Flavonoide, Quercetin, phenolische Benzylamine, Propylgallat, Octylgallat, Dodecylgallat, Butylhydroxyanisol (BHA, E320), Butylhydroxytoluol (BHT, 2,6-Di-tert.-butyl-4-methyl-phenol, E321), Lecithine, Mono- und Diglyceride von Speisefettsäuren verestert mit Citronensäure, Carotinoide, Carotine (z.B. a-Carotin, β-Carotin, Lycopin) und deren Derivate, Phytinsäure, Lactoferrin, EDTA, EGTA), Folsäure und deren Derivate, Ubichinon und Ubichinol und deren Derivate, Ferulasäure und deren Derivate, Zink und dessen Derivate (z.B. ZnO, ZnSO4), Selen und dessen Derivate (z.B. Selenmethionin), Orthophosphate und Na-, Ka- und Ca-Salze der Monophosphorsäure sowie aus Pflanzen isolierte Inhaltsstoffe, Extrakte bzw. Fraktionen davon z.B. aus Tee, Grüntee, Algen, Traubenkernen, Weizenkeimen, Kamille, Rosmarin, Oregano.

Bevorzugte Kühlwirkstoffe sind: 1-Menthol, d-Menthol, racemisches Menthol, Menthonglycerinacetal (Handelsname: Frescolat®MGA), Menthyllactat (Handelsname: Frescolat®ML, vorzugsweise handelt es sich bei Menthyllactat um I-Menthyllactat, insbesondere I-Menthyl-I-lactat), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Besonders bevorzugte Kühlwirkstoffe sind: 1-Menthol, racemisches Menthol, Menthonglycerinacetal (Handelsname: Frescolat®MGA), Menthyllactat (vorzugsweise I-Menthyllactat, insbesondere I-Menthyl-I-lactat, Handelsname:

Frescolat®ML), 3-Menthoxypropan-1,2-diol, 2-Hydroxyethylmenthylcarbonat, 2-Hydroxypropylmenthylcarbonat.

Die Einsatzkonzentration der einzusetzenden Kühlwirkstoffe liegt je nach Substanz vorzugsweise im Konzentrationsbereich von 0,01 bis 20 Gew.-% und bevorzugt im Konzentrationsbereich von 0,1 bis 5 Gew.-%, bezogen auf die Gesamtmasse der fertigen (anwendungsbereiten) erfindungsgemäßen Produkte, insbesondere Nahrungsmittel-, Genussmittel-, Getränke-, Halbfertig-, Mundhygiene-, kosmetische oder pharmazeutische Produkte.

Die erfindungsgemäßen Produkte können beispielsweise folgende Farbstoffe, Färbemittel oder Pigmente enthalten: Lactoflavin (Riboflavin), beta-Carotin, Riboflavin-5'-phosphat, alpha-Carotin, gamma-Carotin, Cantaxanthin, Erythrosin, Kurkumin, Chinolingelb, Gelborange S, Tartrazin, Bixin, Norbixin (Annatto, Orlean), Capsanthin, Capsorubin, Lycopin, beta-Apo-8'-Carotinal, beta-Apo-8'-Carotinsäureethylester, Xantophylle (Flavoxanthin, Lutein, Kryptoxanthin, Rubixanthin, Violaxanthin, Rodoxanthin), Echtes Karmin (Karminsäure, Cochenille), Azorubin, Cochenillerot A (Ponceau 4 R), Beetenrot, Betanin, Anthocyane, Amaranth, Patentblau V, Indigotin I (Indigo-Karmin), Chlorophylle, Kupferverbindungen der Chlorophylle, Brillantsäuregrün BS (Lisamingrün), Brillantschwarz BN, Carbo medicinalis vegetabilis, Titandioxid, Eisenoxide und -hydroxide, Calciumcarbonat, Aluminium, Silber, Gold, Rubinpigment BK (Litholrubin BK), Methylviolett B, Viktoriablau R, Viktoriablau B, Acilanbrillantblau FFR (Brillantwollblau FFR), Naphtolgrün B, Acilanechtgrün 10 G (Alkaliechtgrün 10 G), Ceresgelb GRN, Sudanblau II, Ultramarin, Phtalocyaninblau, Phtalocyaningrün, Echtsäureviolett R. Es können weitere, natürlich gewonnene Extrakte (z.B. Paprikaextrakt, Schwarzmöhrenextrakt, Rotkohlextrakt) zu Färbezwecken verwendet werden. Gute Ergebnisse wurden auch erzielt mit den im Folgenden genannten Farben, den sogenannten Aluminium Lakes: FD & C Yellow 5 Lake, FD & C Blue 2 Lake, FD & C Blue 1 Lake, Tartrazine Lake, Quinoline Yellow Lake, FD & C Yellow 6 Lake, FD & C Red 40 Lake, Sunset Yellow Lake, Carmoisine Lake, Amaranth Lake, Ponceau 4R Lake, Erythrosyne Lake, Red 2G Lake, Allura Red Lake, Patent Blue V Lake, Indigo Carmine Lake, Brilliant Blue Lake, Brown HT Lake, Black PN Lake, Green S Lake und deren Mischungen.

Geeignete (mineralische) Füllstoffe zur Einarbeitung in die erfindungsgemäßen Produkte sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen.

Die weiteren üblichen Grund-, Hilfs- und/oder Zusatzstoffe für erfindungsgemäße Produkte, insbesondere Nahrungsmittel-, Genussmittel-, Getränke-, Halbfertig-, Mundhygiene-, kosmetische oder pharmazeutische Produkte, können in der Regel in Mengen von 0,00001 bis 99,9 Gew.-%, vorzugsweise 10 bis 80 Gew.-%, bezogen auf das Gesamtgewicht der Produktzubereitung, enthalten sein. Ferner können die Zubereitungen Wasser oder andere Lösungsmittel in einer Menge bis zu 99,9 Gew.-%, vorzugsweise 5 bis 80 Gew.-%, bezogen auf das Gesamtgewicht der Produktzubereitung, aufweisen

Gemäß einer weiteren bevorzugten Ausführungsform werden die erfindungsgemäß einzusetzenden Geschmack- und/oder Duftstoffe vor deren Verwendung bei der Herstellung der erfindungsgemäßen Produkte zunächst in eine für Nahrungsmittel-, Genussmittel-, Getränke-, Halbfertig-, Mundhygiene-, kosmetische oder pharmazeutische Produkte geeignete Matrix (Trägerstoff) eingearbeitet, z.B. in Form von Emulsionen, Liposomen, z.B. ausgehend von Phosphatidylcholin, Microsphären, Nanosphären oder auch in Kapseln, Granulaten oder Extrudaten. Besonders bevorzugt wird die Matrix dabei jeweils so gewählt, dass die Geschmack- und/oder Aromastoffe verzögert von der Matrix freigegeben werden, so dass eine langanhaltende Wirkung erzielt wird.

Bevorzugte Matrices, in welche die Geschmack- und/oder Duftstoffe vor deren Verwendung bei der Herstellung der erfindungsgemäßen Zubereitungen eingearbeitet werden, umfassen dabei vorzugsweise ein oder mehrere Materialien ausgewählt aus der folgenden Gruppe: Kohlenhydratpolymere (Polysaccharide) (z.B. Stärke, Stärkederivate, Cellulose oder Cellulosederivate (z.B. Hydroxypropylcellulose), Alginate, Gellan Gum, Agar oder Carragheen), natürliche Fette, natürliche Wachse (z.B. Bienenwachs, Carnaubawachs), Proteine, z.B. Gelatine, Komplexbildner (z.B. Cyclodextrine oder Cyclodextrinderivate, bevorzugt beta-Cyclodextrin).

Es hat sich ferner als vorteilhaft erwiesen die Geschmack- und/oder Duftstoffe vor deren Verwendung bei der Herstellung der erfindungsgemäßen Produkte in eine sprühgetrocknete Form zu überführen. Als Matrices für die erfindungsgemäß einzusetzenden Geschmack- und/oder Duftstoffe in sprühgetrockneter Form können Einzelsubstanzen bzw. Substanzgemische eingesetzt werden. Vorteilhafte Trägerstoffe sind Kohlenhydrate und/oder Kohlenhydratpolymere (Polysaccharide). Als bevorzugte Trägerstoffe für die Geschmack- und/oder Duftstoffe in sprühgetrockneter Form sind zu nennen: Hydrokolloide wie Stärken, abgebaute Stärken, chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate (z.B. Na-Alginat), Pektin, Inulin oder Xanthan Gum zu nennen. Bevorzugte Trägerstoffe sind Maltodextrine sowie Mischungen von Maltodextrinen und Gummi Arabicum, wobei jeweils Maltodextrine mit DE-Werten im Bereich 15 bis 20 wiederum vorteilhaft sind. Der Zersetzungsgrad der Stärke wird mit der Kennzahl "Dextrose-Equivalent" (DE) gemessen, welche die Grenzwerte 0 für das langkettige Glucosepolymer und 100 für die reine Glucose annehmen kann. Die Einkapselung von Geschmack- und/oder Duftstoffen mittels Sprühtrocknung ist dem Fachmann bekannt, und beispielsweise in US 3,159,585, US 3,971,852, US 4,532,145 oder US 5,124,162 beschrieben. Sprühgetrocknete Aromen sind in vielen verschiedenen Geschmacksrichtungen und Partikelgrößen kommerziell erhältlich.

Im Weiteren gelten für die erfindungsgemäßen Verwendungen der erfindungsgemäßen Aromakonzentrate sowie für die erfindungsgemäßen alternativen Anreicherungsverfahren die bereits zu den erfindungsgemäßen Adsorptions- und/oder Desorptionsverfahren gemachten Angaben inklusive der bevorzugten Ausgestaltungen.

Die nachfolgenden Beispiele erläutern die Erfindung, ohne dabei die Erfindung lediglich auf diese Ausführungsbeispiele zu beschränken.

## 1. Erfindungsgemäßes Adsorptionsverfahren am Beispiel einer wässrigen Orangenaromas

Innerhalb des sensorischen Profils des wässrigen Orangenaromas werden 2E-Hexenal (log Pow 1,61), Linalool (log Pow 3,28), Octanal (log Pow 3,03) und Citral (log Pow 3,17) als Geschmacks- und/oder Duftstoffe aus der Gruppe i), die einen positiven sensorischen Eindruck vermitteln, wahrgenommen. Als Geschmacks- und/oder Duftstoffe aus der Gruppe ii), die einen negativen sensorischen Eindruck in der Orangenlösung vermitteln, werden 1-Butanol (log Pow 0,88), 2-Methylbutanol (log Pow 1,29) und Diacetyl (log Pow -1,33) wahrgenommen.

Die Konzentrationen der vorstehend dargestellten Geschmacks- und/oder Duftstoffe in diesem Oragenaroma werden in Tabelle 3 in der Kategorie "wässrige Aroma" in [ppm] angegeben.

Zwei Liter einer solchen Orangenlösung wurde mit Ethanol (96,5-%ig) auf einen Ethanolanteil von 2 Vol.-% eingestellt, so dass die log Pow*-Werte der ein oder mehreren Geschmacks- und/oder Duftstoffe aus der Gruppe i) mindestens einen log Pow*-Wert von 1,49 aufwiesen.

Als Adsorptionsmedium wurde ein mit Divinylbenzol vernetztes Polystyrol verwendet. Als Säule wurde ein zylindrischer Stahlkörper verwendet, der von beiden Seiten mit jeweils einer Schraubkappe verschlossen war. In den beiden Schraubkappen war jeweils ein Schlauchabgang angebracht, durch den die Flüssigkeiten in die Säule hinein bzw. aus der Säule herausgepumpt werden konnten. Das Säulenvolumen betrug 30 ml.

Als Strömungsgeschwindigkeit während des Adsorptionsvorgangs in Schritt e) des erfindungsgemäßen Adsorptionsverfahrens wurde eine Geschwindigkeit von 0,5 cm/sec eingestellt.

Die Temperatur der wässrigen Orangenlösung in Schritt e) des erfindungsgemäßen Adsorptionsvorgangs lag im Bereich von 21 bis 23 °C.

Der Gegendruck auf die wässrige Orangenlösung in Schritt e) des erfindungsgemäßen Adsorptionsverfahrens lag im Bereich von 0,5 bis 0,9 bar

Als Desorptionsmittel wurde reiner Ethanol (96,5 Vol.-%) verwendet.

Die Konzentration der vorstehend genannten Geschmacks- und/oder Duftstoffe werden in Tabelle 3 in der Spalte "Aromakonzentrat" in [ppm] dargestellt.

Hieraus lässt sich leicht erkennen, dass die Anreicherung von Linalool, 2E-Hexenal, Octanal, Geranial und Neral (Gemisch aus Geranial und Neral wird auch Citral genannt) als Geschmacks- und/oder Duftstoffe aus der Gruppe i) im Vergleich zu 2-Methylbutanol, Butylalkohol und Diacetyl als Geschmacks- und/oder Duftstoffe aus der Gruppe ii) mindestens um den Faktor 1,7 erhöht angereichert vorliegen.

## 2. Erfindungsgemäßes Desorptionsverfahren auf Basis einer wässrigen Apfelaromas

Eingesetzt wurde ein wässriges Apfelaroma, das folgende Geschmacks- und/oder Duftstoffe umfasst:

Geschmacks- und/oder Duftstoffe, die einen positiven sensorischen Eindruck vermitteln: 2E-Hexenol, 2E-Hexenal, Butylacetat, Ethylbutyrat, Ethylmethylbutyrat-2 und Hexylacetat;

Geschmacks- und/oder Duftstoffe, die einen negativen sensorischen Eindruck vermitteln: Butanol, 2-Methylbutanol und 3-Methylbutanol.

Der Lösungsmittelanteil in dem wässrigen Aroma war deutlich kleiner als 1 Vol.-% und lag bei etwa 0,1 % (detektiert bei HPLC)

Als Adsorptionsmaterial wurde ein mit Divinylbenzol vernetztes Polystyrol in der schon unter Punkt 1. beschriebenen Säule mit einem Säulenvolumen von 30 ml.

Drei Liter eines wässrigen Apfelaroma wurde gemäß Schritt c) des erfindungsgemäßen Desorptionsverfahrens durch die Säule mit Adsorptionsmaterial durchgeleitet, wobei die vorstehend genannten Geschmacks- und/oder Duftstoffe an das Adsorptionsmaterial adsorbiert wurden, so dass hierbei schon eine Abreicherung polarer Substanzen mit einem log Pow von kleiner 1,30, d.h. ein Verbleiben der polaren Substanzen in dem wässrigen Aroma, zu beobachten ist.

Für den anschließenden Desorptionsvorgang gemäß Schritt e) des erfindungsgemäßen Desorptionsverfahrens wurde ein Ethanol (96,5 Vol.-%) zu Wassergemisch von 1:1 verwendet.

Die Strömungsgeschwindigkeit während des Desorptionsvorgangs in Schritt e) des erfindungsgemäßen Desorptionsverfahrens lag im Bereich von 3 cm/min.

Die Temperatur des Lösungsmittelgemisches lag während des Desorptionsvorgangs in Schritt e) des erfindungsgemäßen Desorptionsverfahrens bei 22°C.

Der Gegendruck in der Vorrichtung, der mit einem Manometer gemessen wurde, lag bei 0,9 bar.

Es wurden die zeitlichen Fraktionen während des Desorptionsvorgangs so geschnitten, das man Fraktionen mit jeweils 5 ml Volumen erhielt.

Die Konzentrationen der einzelnen Geschmacks- und/oder Duftstoffe in den gesammelten Fraktionen 1 bis 6 (Fr. 1 bis Fr. 6) werden in der nachfolgenden Tabelle 4 in [ppm] dargestellt.

Tabelle 4:

|  | Wässriges Aroma | Fr.1 | Fr. 2 | Fr. 3 | Fr. 4 | Fr.5 | Fr. 6 |
|---|---|---|---|---|---|---|---|
| Name | [ppm] | [ppm] | [ppm] | [ppm] | [ppm] | [ppm] | [ppm] |
| 2E-Hexenol | 14,1 | 141,27 | 395,86 | 221,18 | 35,62 | 4,61 | 0,00 |
| 2-Methylbutanol | 7,9 | 50,10 | 81,95 | 17,50 | 0,00 | 0,00 | 0,00 |
| 3-Methylbutanol | 1,2 | 2,91 | 4,01 | 0,00 | 0,00 | 0,00 | 0,00 |
| Butanol | 6,7 | 24,62 | 13,51 | 2,54 | 0,00 | 0,00 | 0,00 |
| 3Z-Hexenol | 2,1 | 6,61 | 58,46 | 42,73 | 0,00 | 0,00 | 0,00 |
| 2E-Hexenal | 3,7 | 3,15 | 15,56 | 88,72 | 86,40 | 45,13 | 16,92 |
| Butylacetat | 25,1 | 2,83 | 184,02 | 669,13 | 802,12 | 488,64 | 205,03 |
| Ethylbutyrat | 6,1 | 0,00 | 23,57 | 136,74 | 152,35 | 76,00 | 30,00 |
| Ethylmethylbutyrat-2 | 0,8 | 0,00 | 3,30 | 14,04 | 17,92 | 12,29 | 6,10 |
| Hexylacetat | 1,3 | 0,00 | 3,46 | 19,84 | 38,14 | 40,08 | 32,41 |

Da 3000 ml eines wässrigen Aromas eingesetzt wurden und man sechs Fraktionen (Fr. 1 bis Fr. 6) mit jeweils 5 ml Volumen erhielt, ergibt sich eine Anreicherung um den Faktor 100, bezogen auf die in allen Fraktionen aufsummierten Konzentrationen der jeweiligen Geschmacks- und/oder Duftstoffe.

Es ist leicht zu erkennen, dass Butanol als eine Verbindung der Gruppe ii), die einen negativen sensorischen Eindruck vermitteln, auf insgesamt 670 ppm aufkonzentriert sein sollte, die Summation der Fraktionen 1 bis 3 jedoch nur 40,5 ppm ergibt. Dies hängt damit zusammen, dass Butanol schon während des Adsorptionsvorganges des beschriebenen Desorptionsverfahrens sehr schlecht and das Adsorptionsmaterial adsorbiert wurde.

Betrachtet man die Verbindung Ethylmethylbutyrat-2, so hätte diese Verbindung als eine Verbindung aus der Gruppe i), die einen positiven sensorischen Eindruck vermittelt, in der Summation aller Fraktionen bei ca. 80 ppm vorliegen sollen. Analytisch findet man jedoch, Fehler der Messmethodik beinhaltend, 54 ppm Ethylmethylbutyrat-2 wieder. Entscheidend hierbei ist jedoch, dass Ethylmethylbutyrat-2 im wesentlichen in den Fraktionen ab Fraktion 3 wiederfindet, also in Fraktionen, in denen Butanol als eine Verbindung, die einen negativen sensorischen Eindruck vermittelt, im wesentlichen nicht mehr vorhanden ist.

**Patentansprüche**

1. Verfahren zur Anreicherung von ein oder mehreren Geschmacks- und/oder Duftstoffen, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma, umfassend oder bestehend aus folgenden Schritten:

a) Bereitstellen eines wässrigen Aromas umfassend

i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,

ii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure.

b) Bereitstellen ein, zwei oder mehrerer lebensmittelgeeigneter Lösungsmittel,

c) Zugabe des oder der Lösungsmittel aus Schritt b) zu der Lösung aus Schritt a), wobei der resultierende Lösungsmittelanteil so eingestellt wird, dass die ein oder mehrere Geschmacks- und/oder Duftstoffe aus der Gruppe i) unabhängig voneinander, jeweils einen log Pow*-Wert von größer oder gleich 1,20 aufweisen und der log Pow*-Wert wie folgt berechnet wird:

$$\log Pow^* = \log Pow - x \cdot P'(x) / \log Pow$$

mit log Pow als dem dekadischen Logarithmus des Verteilungskoeffizienten des Geschmacks- und/oder Duftstoffes zwischen 1-Octanol zu Wasser,

mit x als dem resultierenden Lösungsmittelanteil bezogen auf den Gesamtvolumenanteil, als 1,0 normiert und mit P'(x) als Polaritätsparameter für das entsprechende Lösungsmittel bei dem resultierenden Lösungsmittelanteil x,

d) Bereitstellen eines Adsorptionsmaterials in einer geeigneten Vorrichtung,

e) Durchleiten der Lösung aus Schritt c) durch eine Vorrichtung mit Adsorptionsmaterial aus Schritt d), so dass vorwiegend die ein oder mehreren Geschmacks- und/oder Duftstoffe, die einen positiven sensorischen Eindruck vermitteln, an das Absorptionsmaterial adsorbiert werden,

f) Bereitstellen von ein, zwei oder mehreren lebensmittelgeeigneten Lösungsmitteln und

g) Desorbieren der Geschmacks- und/oder Duftstoffe von dem Adsorptionsmaterial aus Schritt e) mit dem oder den Lösungsmitteln aus Schritt f), so dass die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a).

2. Verfahren gemäß Anspruch 1 zur Herstellung eines Aromakonzentrates, in dem ein oder mehrere Geschmacks- und/oder Duftstoffe, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma höher angereichert sind, umfassend oder bestehend aus folgenden Schritten:

Schritte a) bis f) gemäß Anspruch 1 und

g) Desorbieren der Geschmacks- und/oder Duftstoffe von dem Adsorptionsmaterial aus Schritt e) mit dem oder den Lösungsmitteln aus Schritt f), so dass in dem resultierenden Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a).

3. Verfahren nach Anspruch 2, **dadurch gekennzeichnet, dass** in Schritt c) der Lösungsmittelanteil gleichzeitig so eingestellt wird, dass die ein oder mehreren Geschmacks- und/oder Duftstoffe der Gruppe ii) unabhängig voneinander, jeweils einen log Pow*-Wert von kleiner 1,20 aufweisen.

4. Verfahren nach einem der Ansprüche 2 bis 3, **dadurch gekennzeichnet, dass** in Schritt c) der Lösungsmittelanteil gleichzeitig im Bereich zwischen 1 Vol.-% und 6 Vol.-% bezogen auf die resultierende Lösung liegt.

5. Verfahren nach einem der Ansprüche 2 bis 4, **dadurch gekennzeichnet, dass** das oder die Lösungsmittel aus Schritt b) ausgewählt werden aus der Gruppe bestehend aus Methanol, Ethanol, Propanol und Isopropanol.

6. Verfahren nach einem der Ansprüche 2 bis 5, **dadurch gekennzeichnet, dass** das oder die Lösungsmittel aus Schritt f) ausgewählt werden aus der Gruppe bestehend aus Methanol, Ethanol, Propanol, Isopropanol, Ethylacetat, Diacetin, Triacetin, flüssiges Kohlendioxid, lebensmittelgeeignete Fluorchlorkohlenwasserstoffe und Pflanzentriglyceride.

7. Verfahren nach einem der Ansprüche 2 bis 6, **dadurch gekennzeichnet, dass** der Gegendruck auf die Lösung aus Schritt c) zumindest teilweise während des Adsorptionsvorgangs in Schritt e) im Bereich von 0,1 bar bis 4,0 bar liegt.

8. Verfahren zur Anreicherung von ein oder mehreren Geschmacks- und/oder Duftstoffen, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma, umfassend oder bestehend aus folgenden Schritten:

   a) Bereitstellen eines wässrigen Aromas umfassend

   i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,
   ii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Dimethylsulfid, Methylmercaptan, Milchsäure und Essigsäure,
   wobei der Lösungsmittelanteil bezogen auf das Gesamtvolumen der wässrigen Lösung im Bereich von 0 Vol.-% bis kleiner 1 Vol.-% liegt,

   b) Bereitstellen eines Adsorptionsmaterials in einer geeigneten Vorrichtung,
   c) Durchleitung der Lösung aus Schritt a) durch die Vorrichtung mit Adsorptionsmaterial aus Schritt b), so dass sowohl die ein oder mehreren Geschmacks- und/oder Duftstoffe aus der Gruppe i) als auch die ein oder mehreren Geschmacks- und/oder Duftstoffe aus der Gruppe ii) an das Absorptionsmaterial adsorbiert werden,
   d) Bereitstellen eines oder mehrerer Lösungsmittel oder Lösungsmittelgemische mit Wasser, wobei die Lösungsmittel oder die Lösungsmittelgemische so gewählt werden, dass ein oder mehrere Geschmacks- und/oder Duftstoffe der Gruppe ii) einen log Pow*-Wert von 1,20 oder kleiner aufweisen und
   e) Desorbieren der Geschmacks- und/oder Duftstoffe von dem Adsorptionsmaterial aus Schritt c) mit den Lösungsmitteln oder den Lösungsmittelgemischen aus Schritt d), wobei ein, zwei, drei, vier, fünf, sechs, sieben oder mehrere Fraktionen über die Zeit gesammelt werden und in einer dieser Fraktionen oder in einer Mischung aus zwei, drei, vier, fünf, sechs, sieben oder mehreren dieser Fraktionen die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a), wobei das zur Desorption verwendete oder die zur Desorption verwendeten Lösungsmittel so gewählt werden, dass die zu desorbierenden Geschmacks- und/oder Duftstoffe einen log Pow* Wert von 1,20 oder kleiner besitzen und der log Pow*-Wert wie folgt berechnet wird:

$$\log Pow^* = \log Pow - x * P'(x) / \log Pow$$

mit log Pow als dem dekadischen Logarithmus des Verteilungskoeffizienten des Geschmacks- und/oder Duftstoffes zwischen 1-Octanol zu Wasser,
mit x als dem resultierenden Lösungsmittelanteil bezogen auf den Gesamtvolumenanteil, als 1,0 normiert und
mit P'(x) als Polaritätsparameter für das entsprechende Lösungsmittel bei dem resultierenden Lösungsmittelanteil x.

9. Verfahren gemäß Anspruch 8 zur Herstellung eines Aromakonzentrates, in dem ein oder mehrere Geschmacks- und/oder Duftstoffe, die einen positiven sensorischen Eindruck vermitteln, unabhängig voneinander, jeweils um den Faktor 1,5 oder mehr im Vergleich zu ein oder mehreren Geschmacks- und/oder Duftstoffen, die einen negativen sensorischen Eindruck vermitteln, bezogen auf ein wässriges Aroma höher angereichert sind, umfassend oder bestehend aus folgenden Schritten:

Schritte a) bis d) gemäß Anspruch 8 und

e) Desorbieren der Geschmacks- und/oder Duftstoffe von dem Adsorptionsmaterial aus Schritt c) mit den Lösungsmitteln oder den Lösungsmittelgemischen aus Schritt d), wobei ein, zwei, drei, vier, fünf, sechs, sieben oder mehrere Fraktionen über die Zeit gesammelt werden und eine dieser Fraktion oder eine Mischung aus zwei, drei, vier, fünf, sechs, sieben oder mehreren dieser Fraktionen das Aromakonzentrat bilden, wobei in dem resultierenden Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert vorliegen als in dem wässrigen Aroma aus Schritt a), wobei das zur Desorption verwendete oder die zur Desorption verwendeten Lösungsmittel so gewählt werden, dass die zu desorbierenden Geschmacks- und/oder Duftstoffe einen log Pow* Wert von 1,20 oder kleiner besitzen und der log Pow*-Wert wie folgt berechnet wird:

$$\log P_{ow}* = \log P_{ow} - x * P'(x) / \log P_{ow}$$

mit log Pow als dem dekadischen Logarithmus des Verteilungskoeffizienten des Geschmacks- und/oder Duftstoffes zwischen Octanol zu Wasser,
mit x als dem resultierenden Lösungsmittelanteil bezogen auf den Gesamtvolumenanteil, als 1,0 normiert und
mit P'(x) als Polaritätsparameter für das entsprechende Lösungsmittel bei dem resultierenden Lösungsmittelanteil x.

10. Verfahren nach Anspruch 9, **dadurch gekennzeichnet, dass** der Gegendruck auf die Lösung aus Schritt d) zumindest teilweise während des Desorptionsvorgangs in Schritt e) im Bereich von 0,1 bar bis 4,0 bar liegt.

11. Verfahren nach einem der Ansprüche 2- 7, 9 oder 10, **dadurch gekennzeichnet, dass** das Adsorptionsmaterial ausgewählt wird aus der Gruppe bestehend aus vernetzten Polystyrolen, bevorzugt Ethylvinylbenzol und Divinylbenzol, Vinylpyrrolidin und Divinylbenzol, Vinylpyridin und Divinylbenzol und Styrol und Divinylbenzol; und andere Polymeren, bevorzugt Polyaromate, Polystyrole, Polymethacrylate, Polypropylene, Polyester und Polytetrafluorethylen.

12. Aromakonzentrat herstellbar nach einem Verfahren gemäß einer der Ansprüche 1 bis 11, wobei das resultierende Aromakonzentrat die ein oder mehreren Geschmacks- und/oder Duftstoffe aus der Gruppe i) im Vergleich zu den ein oder mehreren Geschmacks- und/oder Duftstoffen aus der Gruppe ii) unabhängig voneinander, jeweils um den Faktor 1,5 oder mehr höher angereichert umfasst als das wässrige Aroma und

wobei im Schritt a) des Verfahrens nach einem der Ansprüche 1 bis 11 ein wässriges Aroma bereit gestellt wird, umfassend

i. ein oder mehrere Geschmacks- und/oder Duftstoffe, die einen positiven sensorischen Eindruck vermitteln, ausgewählt aus der Liste bestehend aus Ethylbutyrat, Ethylmethylbutyrat-2, Methylcapronat, Linalool, alpha-Ionon, beta-Ionon, delta-Decalacton, 2E-Hexenol, 2E-Hexenal, Hexanal, beta-Damascenon, Octanal, Nootkaton, p-Menthenthiol-1,8, Benzaldehyd, gamma-Decalacton, Linalooloxid, Furfurylthiol-2, 4-Vinylguaiacol, isomere Isopropylmethoxypyrazine, isomere Ethyldimethylpyrazine, Indol, Methyljasmonat, Jasminlacton, Dipropyldisulfid, Dipropyltrisulfid, Methypropyldisulfid, L-Menthol, Menthon, L-Carvon, Isoamylacetat, 2-Acetyl-1-pyrrolin, 2E,4Z-Decadienal, 3,5-Dimethyltrithiolan, Citral, Caryophyllen, 1-Octen-3-ol, 1-Octen-3-on, Hydroxybenzylaceton, cis-3-Hexenol, 3Z-Hexenol, Methylbutyrat, Geraniol, Ethyl-2E,4Z-decadienoat, 8-Mercapto-p-Menth-1-en-3-on, 2E,4Z,7Z-Tridecatrienal, 2E,5Z-Undecadienal, Nonanal, 4-Ocanolid, 5-Octanolid, Phenylethanol, Weinlacton und Menthofurolactone,
ii. ein oder mehrere Geschmacks- und/oder Duftstoffe, die einen negativen sensorischen Eindruck vermitteln, ausgewählt aus der Liste bestehend aus $C_1$-$C_5$-Alkohole, bevorzugt, Methanol, Ethanol, Propanol, Isopropanol, Butanol, 2-Methylbutanol, 3-Methylbutanol, Diacetyl, Acetaldehyd, Furfural, Furfurylalkohol, Phenol, Acetoin, Methylmercaptan und Milchsäure.

13. Nahrungsmittel-, Genussmittel-, Getränke-, Halbfertig-, Mundhygiene-, kosmetische oder pharmazeutische Produkte umfassend ein oder mehrere Aromakonzentrate gemäß Anspruch 12 sowie ein oder mehrere Grund-, Hilfs- und/ oder Zusatzstoffe.

14. Verwendung von Aromakonzentraten gemäß Anspruch 12 in Nahrungsmittel-, Genussmittel-, Getränke-, Halbfertig-, Mundhygiene-, kosmetischen oder pharmazeutischen Produkten.

15. Verwendung von Aromakonzentraten gemäß Anspruch 12 zur Herstellung von Nahrungsmittel-, Genussmittel-, Getränke-, Halbfertig-, Mundhygiene-, kosmetischen oder pharmazeutischen Produkten.

## Claims

1. A method of concentrating one or more flavour and/or fragrance compounds which impart a positive sensory impression, each independently of one another by a factor of 1.5 or more compared with one or more flavour and/or fragrance compounds which impart a negative sensory impression, based on an aqueous flavouring, comprising or consisting of the following steps:

a) preparation of an aqueous flavouring comprising

i. one or more flavour and/or fragrance compounds which impart a positive sensory impression, selected from the list consisting of ethyl butyrate, ethyl methyl butyrate-2, methyl caproate, linalool, alpha-ionone, beta-ionone, delta-decalactone, 2E-hexenol, 2E-hexenal, hexanal, beta-damascenone, octanal, nootkatone, p-menthenethiol-1,8, benzaldehyde, gamma-decalactone, linalool oxide, 2-furfurylthiol, 4-vinylguaiacol, isomeric isopropyl methoxypyrazines, isomeric ethyl dimethyl pyrazines, indole, methyl jasmonate, jasmine lactone, dipropyl disulfide, dipropyl trisulfide, methyl propyl disulfide, L-menthol, menthone, L-carvone, isoamyl acetate, 2-acetyl-1-pyrroline, 2E,4Z-decadienal, 3,5-dimethyltrithiolane, citral, caryophyllene, 1-octen-3-ol, 1-octen-3-one, hydroxybenzyl acetone, cis-3-hexenol, 32-hexenol, methyl butyrate, geraniol, ethyl-2E,4Z-decadienoate, 8-mercapto-p-menth-1-en-3-one, 2E,4Z,7Z-tridecatrienal, 2E,5Z-undecadienal, nonanal, 4-octanolide, 5-octanolide, phenylethanol, wine lactone and menthofurolactones,
ii. one or more flavour and/or fragrance compounds which impart a negative sensory impression, selected from the list consisting of $C_1$-$C_5$ alcohols, preferably methanol, ethanol, propanol, isopropanol, butanol, 2-methylbutanol, 3-methylbutanol, diacetyl, acetaldehyde, furfural, furfuryl alcohol, phenol, acetoin, dimethyl sulfide, methyl mercaptan, lactic acid and acetic acid,

b) preparation of one, two or more food-safe solvents,
c) addition of the solvent or solvents from step b) to the solution from step a), the resulting proportion of solvent being adjusted so that the one or more flavour and/or fragrance compounds from group i) each independently of one another have a log Pow* value of greater than or equal to 1.20 and the log Pow* value is calculated as follows:

$$\log Pow^* = \log Pow - x*P'(x)/\log Pow$$

with log Pow as the decimal logarithm of the distribution coefficient of the flavour and/or fragrance compound between 1-octanol and water,
with x as the resulting proportion of solvent based on the total proportion by volume, standardised as 1.0 and
with P'(x) as polarity parameter for the corresponding solvent with the resulting proportion of solvent x,
d) preparation of an adsorption material in a suitable apparatus,
e) passing of the solution from step c) through an apparatus with adsorption material from step d), so that primarily the one or more flavour and/or fragrance compounds which impart a positive sensory impression are adsorbed on the absorption material,
f) preparation of one, two or more food-safe solvents and

g) desorption of the flavour and/or fragrance compounds from the adsorption material from step e) with the solvent or solvents from step f), so that the one or more flavour and/or fragrance compounds from group i), compared with the one or more flavour and/or fragrance compounds from group ii), are present each independently of one another in a higher concentration by a factor of 1.5 or more than in the aqueous flavouring from step a).

2. A method according to claim 1 for the production of a flavouring concentrate, in which one or more flavour and/or fragrance compounds which impart a positive sensory impression are concentrated each independently of one another by a factor of 1.5 or more compared with one or more flavour and/or fragrance compounds which impart a negative sensory impression, based on an aqueous flavouring, comprising or consisting of the following steps:

steps a) to f) according to claim 1 and

g) desorption of the flavour and/or fragrance compounds from the adsorption material from step e) with the solvent or solvents from step f), so that in the resulting flavouring concentrate the one or more flavour and/or fragrance compounds from group i), each independently of one another, compared with the one or more flavour and/or fragrance compounds from group ii), are present in a higher concentration by a factor of 1.5 or more than in the aqueous flavouring from step a).

3. The method according to claim 2, **characterised in that** in step c) the proportion of solvent is at the same time adjusted so that the one or more flavour and/or fragrance compounds from group ii) each independently of one another have a log Pow* value of less than 1.20.

4. The method according to one of claims 2 to 3, **characterised in that** in step c) the proportion of solvent is at the same time in the range between 1 vol.% and 6 vol.%, based on the resulting solution.

5. The method according to one of claims 2 to 4, **characterised in that** the solvent or solvents from step b) are selected from the group consisting of methanol, ethanol, propanol and isopropanol.

6. The method according to one of claims 2 to 5, **characterised in that** the solvent or solvents from step f) are selected from the group consisting of methanol, ethanol, propanol, isopropanol, ethyl acetate, diacetin, triacetin, liquid carbon dioxide, food-safe chlorofluorocarbons and vegetable triglycerides.

7. The method according to one of claims 2 to 6, **characterised in that** the counter pressure on the solution from step c) is in the range from 0.1 bar to 4.0 bar during at least part of the adsorption operation in step e).

8. A method of concentrating one or more flavour and/or fragrance compounds which impart a positive sensory impression each independently of one another by a factor of 1.5 or more compared with one or more flavour and/or fragrance compounds which impart a negative sensory impression, based on an aqueous flavouring, comprising or consisting of the following steps:

a) preparation of an aqueous flavouring comprising

i. one or more flavour and/or fragrance compounds which impart a positive sensory impression, selected from the list consisting of ethyl butyrate, ethyl methyl butyrate-2, methyl caproate, linalool, alpha-ionone, beta-ionone, delta-decalactone, 2E-hexenol, 2E-hexenal, hexanal, beta-damascenone, octanal, nootkatone, p-menthenethiol-1,8, benzaldehyde, gamma-decalactone, linalool oxide, 2-furfurylthiol, 4-vinylguaiacol, isomeric isopropyl methoxypyrazines, isomeric ethyl dimethyl pyrazines, indole, methyl jasmonate, jasmine lactone, dipropyl disulfide, dipropyl trisulfide, methyl propyl disulfide, L-menthol, menthone, L-carvone, isoamyl acetate, 2-acetyl-1-pyrroline, 2E,4Z-decadienal, 3,5-dimethyltrithiolane, citral, caryophyllene, 1-octen-3-ol, 1-octen-3-one, hydroxybenzyl acetone, cis-3-hexenol, 3Z-hexenol, methyl butyrate, geraniol, ethyl-2E,4Z-decadienoate, 8-mercapto-p-menth-1-en-3-one, 2E,4Z,7Z-tridecatrienal, 2E,5Z-undecadienal, nonanal, 4-octanolide, 5-octanolide, phenylethanol, wine lactone and menthofurolactones,
ii. one or more flavour and/or fragrance compounds which impart a negative sensory impression, selected from the list consisting of $C_1$-$C_5$ alcohols, preferably methanol, ethanol, propanol, isopropanol, butanol, 2-methylbutanol, 3-methylbutanol, diacetyl, acetaldehyde, furfural, furfuryl alcohol, phenol, acetoin, dimethyl sulfide, methyl mercaptan, lactic acid and acetic acid,
wherein the proportion of solvent is in the range of 0 vol.% to less than 1 vol.%, based on the total volume of the aqueous solution,

b) preparation of an adsorption material in a suitable apparatus,

c) passing of the solution from step a) through the apparatus with adsorption material from step b) so that both the one or more flavour and/or fragrance compounds from group i) and the one or more flavour and/or fragrance compounds from group ii) are adsorbed on the absorption material,

d) preparation of one or more solvents or solvent mixtures with water, the solvent or solvent mixtures being selected so that one or more flavour and/or fragrance compounds from group ii) have a log Pow* value of 1.20 or less and

e) desorption of the flavour and/or fragrance compounds from the adsorption material from step c) with the solvents or the solvent mixtures from step d), wherein one, two, three, four, five, six, seven or more fractions are collected over time and in one of these fractions or in a mixture of two, three, four, five, six, seven or more of these fractions, the one or more flavour and/or fragrance compounds from group i), are present each independently of one another in a higher concentration by a factor of 1.5 or more, compared with the one or more flavour and/or fragrance compounds from the group ii), than in the aqueous flavouring from step a), wherein the solvent or solvents used for the desorption are selected so that the flavour and/or fragrance compounds to be desorbed have a log Pow* value of 1.20 or less and the log Pow* value is calculated as follows:

$$\log \text{Pow*} = \log \text{Pow} - x * P'(x) / \log \text{Pow}$$

with log Pow as the decimal logarithm of the distribution coefficient of the flavour and/or fragrance compound between 1-octanol and water,

with x as the resulting proportion of solvent based on the total proportion by volume, standardised as 1.0 and

with P'(x) as polarity parameter for the corresponding solvent with the resulting proportion of solvent x.

9. The method according to claim 8 for the production of a flavouring concentrate in which one or more flavour and/or fragrance compounds which impart a positive sensory impression are more highly concentrated each independently of one another by a factor of 1.5 or more compared with one or more flavour and/or fragrance compounds which impart a negative sensory impression, based on an aqueous flavouring, comprising or consisting of the following steps:

steps a) to d) according to claim 8 and

e) desorption of the flavour and/or fragrance compounds from the adsorption material from step c) with the solvents or the solvent mixtures from step d), one, two, three, four, five, six, seven or more fractions being collected over time and one of these fractions or a mixture of two, three, four, five, six, seven or more of these fractions forming the flavouring concentrate, wherein the one or more flavour and/or fragrance compounds from group i) are present in the resulting flavouring concentrate each independently of one another in a higher concentration by a factor of 1.5 or more, compared with the one or more flavour and/or fragrance compounds from group ii), than in the aqueous flavouring from step a), wherein the solvent or solvents used for the desorption are selected so that the flavour and/or fragrance compounds to be desorbed have a log Pow* value of 1.20 or less and the log Pow* value is calculated as follows:

$$\log \text{Pow*} = \log \text{Pow} - x * P'(x) / \log \text{Pow}$$

with log Pow as the decimal logarithm of the distribution coefficient of the flavour and/or fragrance compound between octanol and water,

with x as the resulting proportion of solvent based on the total proportion by volume, standardised as 1.0 and

with P'(x) as polarity parameter for the corresponding solvent with the resulting proportion of solvent x.

10. The method according to claim 9, **characterised in that** the counter-pressure on the solution from step d) is in the range of 0.1 bar to 4.0 bar during at least part of the desorption operation in step e).

11. The method according to one of claims 2 to 7, 9 or 10, **characterised in that** the adsorption material is selected from the group consisting of crosslinked polystyrenes, preferably ethyl vinyl benzene and divinylbenzene, vinyl pyrrolidine and divinylbenzene, vinyl pyridine and divinylbenzene and styrene and divinylbenzene; and other polymers, preferably polyaromatics, polystyrenes, polymethacrylates, polypropylenes, polyesters and polytetrafluorethylene.

**12.** A flavouring concentrate that can be produced by a method according to one of claims 1 to 11, wherein the resulting flavouring concentrate comprises the one or more flavour and/or fragrance compounds from group i), compared with the one or more flavour and/or fragrance compounds from group ii), each independently of one another in a higher concentration by a factor of 1.5 or more than the aqueous flavouring and

wherein, in step a) of the method according to one of claims 1 to 11 an aqueous flavouring is prepared comprising

i. one or more flavour and/or fragrance compounds which impart a positive sensory impression, selected from the list consisting of ethyl butyrate, ethyl methyl butyrate-2, methyl caproate, linalool, alpha-ionone, beta-ionone, delta-decalactone, 2E-hexenol, 2E-hexenal, hexanal, beta-damascenone, octanal, nootkatone, p-menthenethiol-1,8, benzaldehyde, gamma-decalactone, linalool oxide, 2-furfurylthiol, 4-vinylguaiacol, isomeric isopropyl methoxypyrazines, isomeric ethyl dimethyl pyrazines, indole, methyl jasmonate, jasmine lactone, dipropyl disulfide, dipropyl trisulfide, methyl propyl disulfide, L-menthol, menthone, L-carvone, isoamyl acetate, 2-acetyl-1-pyrroline, 2E,4Z-decadienal, 3,5-dimethyltrithiolane, citral, caryophyllene, 1-octen-3-ol, 1-octen-3-one, hydroxybenzyl acetone, cis-3-hexenol, 3Z-hexenol, methyl butyrate, geraniol, ethyl-2E,4Z-decadienoate, 8-mercapto-p-menth-1-en-3-one, 2E,4Z,7Z-tridecatrienal, 2E,5Z-undecadienal, nonanal, 4-octanolide, 5-octanolide, phenylethanol, wine lactone and menthofurolactones,

ii. one or more flavour and/or fragrance compounds which impart a negative sensory impression, selected from the list consisting of $C_1$-$C_5$ alcohols, preferably, methanol, ethanol, propanol, isopropanol, butanol, 2-methylbutanol, 3-methylbutanol, diacetyl, acetaldehyde, furfural, furfuryl alcohol, phenol, acetoin, methyl mercaptan and lactic acid.

**13.** Food products, semi-luxury food and tobacco products, drinks products, semi-finished products, oral hygiene products, cosmetic or pharmaceutical products comprising one or more flavouring concentrates according to claim 12 and one or more raw materials, auxiliary substances and/or additives.

**14.** Use of flavouring concentrates according to claim 12 in food products, semi-luxury food and tobacco products, drinks products, semi-finished products, oral hygiene products, cosmetic or pharmaceutical products.

**15.** Use of flavouring concentrates according to claim 12 for the production of food products, semi-luxury food and tobacco products, drinks products, semi-finished products, oral hygiene products, cosmetic or pharmaceutical products.

**Revendications**

**1.** Procédé pour l'enrichissement d'un ou de plusieurs agents de sapidité et/ou matières odoriférantes qui communiquent une impression sensorielle positive, indépendamment l'un de l'autre, respectivement d'un facteur de 1,5 ou plus en comparaison avec un ou plusieurs agents de sapidité et/ou matières odoriférantes qui communiquent une impression sensorielle négative, rapporté à un arôme aqueux, comportant ou consistant en les étapes suivantes :

a) mise à disposition d'un arôme aqueux, comprenant

i. uns ou plusieurs agents de sapidité et/ou matières odoriférantes qui communiquent une impression sensorielle positive, choisis parmi la liste consistant en le butyrate d'éthyle, l'éthylméthylbutyrate-2, le capronate de méthyle, le linalool, l'alpha-ionone, la bêta-ionone, la delta-décalactone, le 2E-hexénol, le 2E-hexénal, l'hexanal, la bêta-damascénone, l'octanal, la nootcatone, le p-menthénethiol-1,8, le benzaldéhyde, la gamma-décalactone, l'oxyde de linalool, le furfurylthiol-2, le 4-vinylgaïacol, l'isopropylméthoxypyrazine isomère, l'éthyldiméthylpyrazine isomère, l'indol, le méthyljasmonate, la jasminolactone, le disulfure de dipropyle, le trisulfure de dipropyle, le disulfure de méthypropyle, le L-menthol, la menthone, la L-carvone, l'acétat d'isoamyle, la 2-acétyl-1-pyrroline, le 2E,4Z-décadiénal, le 3,5-diméthyltrithiolane, le citral, le caryophyllène, l'1-octène-3-ol, l'1-octène-3-one, l'hydroxybenzylacétone, le cis-3-hexénol, le 3Z-hexénol, le butyrate de méthyle, le géraniol, l'éthyl-2E,4Z-décadiénoate, la 8-mercapto-p-menth-1-én-3-one, le 2E,4Z,7Z-tridécatriénal, le 2E,5Z-undécadiénal, le nonanal, le 4-octanolide, le 5-octanolide, le phényléthanol, la lactone des vins et la menthofurolactone,

ii. un ou plusieurs agents de sapidité et/ou matières odoriférantes qui communiquent une impression sensorielle négative, choisis parmi la liste consistant en des alcools en $C_1$ à $C_5$, de préférence le méthanol, l'éthanol, le propanol, l'isopropanol, le butanol, le 2-méthylbutanol, le 3-méthylbutanol, le diacétyle, l'acétaldéhyde, le furfural, l'alcool furfurylique, le phénol, l'acétoïne, le sulfure de diméthyle, le méthylmercaptan, l'acide lactique et l'acide acétique.

b) mise à disposition d'un, de deux ou de trois solvants alimentaires,

c) addition du ou des solvants provenant de l'étape b) à la solution provenant de l'étape a), la proportion de solvant résultante étant réglée de telle sorte que lesdits un ou plusieurs agents de sapidité et/ou matières odoriférantes du groupe i), indépendamment les uns des autres, présentent chacun une valeur log pow* supérieure ou égale à 1,20, la valeur log pow* étant calculée comme suit :

$$\log pow^* = \log pow - x^* \, P'(x) \, / \log pow$$

avec log pow comme logarithme décimal du coefficient de répartition de l'agent de sapidité et/ou de la matière odoriférante entre le 1-octanol et l'eau,

avec x comme la proportion résultante de solvant rapportée à la part totale en volume, normée à 1,0, et

avec P'(x) comme le paramètre de polarité pour le solvant correspondant pour la proportion résultante x du solvant,

d) mise à disposition d'un matériau d'adsorption dans un dispositif approprié,

e) acheminement de la solution provenant de l'étape c) à travers un dispositif avec du matériau d'adsorption provenant de l'étape d), de telle sorte que lesdits un ou plusieurs agents de sapidité et/ou matières odoriférantes qui communiquent un effet sensoriel positif soient adsorbés en priorité sur le matériau d'adsorption,

f) mise à disposition d'un, de deux ou de plusieurs solvants alimentaires et

g) désorption des agents de sapidité et/ou des matières odoriférantes du matériau d'adsorption provenant de l'étape e) par le ou les solvants provenant de l'étape f), de telle sorte que lesdits un ou plusieurs agents de sapidité et/ou matières odoriférantes provenant du groupe i), en comparaison avec lesdits un ou plusieurs agents de sapidité et/ou matières odoriférantes provenant du groupe ii), indépendamment les uns des autres, se trouvent plus enrichis, respectivement d'un facteur de 1,5 ou plus, que dans l'arôme aqueux provenant de l'étape a).

**2.** Procédé selon la revendication 1 pour la préparation d'un concentré d'arômes, dans lequel un ou plusieurs agents de sapidité et/ou matières odoriférantes qui communiquent une impression sensorielle positive, indépendamment les uns des autres, sont plus enrichis respectivement d'un facteur de 1,5 ou plus en comparaison avec un ou plusieurs agents de sapidité et/ou matières odoriférantes qui communiquent une impression sensorielle négative, rapporté à un arôme aqueux, comportant ou consistant en les étapes suivantes :

étapes a) à f) selon la revendication 1 et

g) désorption des agents de sapidité et/ou matières odoriférantes du matériau d'adsorption provenant de l'étape e) avec le ou les solvants provenant de l'étape f), de telle sorte que dans le concentré d'arômes résultant, lesdits un ou plusieurs agents de sapidité et/ou matières odoriférantes du groupe i), indépendamment les uns des autres, se trouvent plus enrichis d'un facteur de 1,5 ou plus en comparaison avec lesdits un ou plusieurs agents de sapidité et/ou matières odoriférantes du groupe ii), que dans l'arôme liquide provenant de l'étape a).

**3.** Procédé selon la revendication 2, **caractérisé en ce que** dans l'étape c), la proportion du solvant est ajustée en même temps de telle sorte que lesdits un ou plusieurs agents de sapidité et/ou matières odoriférantes du groupe ii), indépendamment les uns des autres, présentent respectivement une valeur log pow* de moins de 1,20.

**4.** Procédé selon l'une quelconque des revendications 2 ou 3, **caractérisé en ce qu'**à l'étape c), la proportion de solvant se trouve en même temps dans l'intervalle entre 1 % en volume et 6 % en volume, rapportée à la solution résultante.

**5.** Procédé selon l'une quelconque des revendications 2 à 4, **caractérisé en ce que** le ou les solvants provenant de l'étape b) sont choisis parmi le groupe consistant en le méthanol, l'éthanol, le propanol et l'isopropanol.

**6.** Procédé selon l'une quelconque des revendications précédentes 2 à 5, **caractérisé en ce que** le ou les solvants provenant de l'étape f) sont choisis parmi le groupe consistant en le méthanol, l'éthanol, le propanol, l'isopropanol, l'acétate d'éthyle, la diacétine, la triacétine, le dioxyde de carbone liquide, les hydrocarbures fluochlorés alimentaires et les triglycérides végétaux.

**7.** Procédé selon l'une quelconque des revendications 2 à 6, **caractérisé en ce que** la contre-pression sur la solution de l'étape c) se trouve au moins partiellement pendant le processus d'adsorption à l'étape e) dans l'intervalle de 0,1 à 4,0 bar.

**8.** Procédé pour l'enrichissement d'un ou de plusieurs agents de sapidité et/ou matières odoriférantes qui communiquent une impression sensorielle positive, indépendamment l'un de l'autre, respectivement d'un facteur de 1,5 ou plus en comparaison avec un ou plusieurs agents de sapidité et/ou matières odoriférantes qui communiquent une impression sensorielle négative, rapporté à un arôme aqueux, comportant ou consistant en les étapes suivantes :

a) mise à disposition d'un arôme aqueux, comprenant

i. un ou plusieurs agents de sapidité et/ou matières odoriférantes qui communiquent une impression sensorielle positive, choisis parmi la liste consistant en le butyrate d'éthyle, l'éthylméthylbutyrate-2, le capronate de méthyle, le linalool, l'alpha-ionone, la bêta-ionone, la delta-décalactone, le 2E-hexénol, le 2E-hexénal, l'hexanal, la bêta-damascénone, l'octanal, la nootcatone, le p-menthènethiol-1,8, le benzaldéhyde, la gamma-décalactone, l'oxyde de linalool, le furfurylthiol-2, le 4-vinylgaïacol, l'isopropylméthoxypyrazine isomère, l'éthyldiméthylpyrazine isomère, l'indol, le méthyljasmonate, la jasminolactone, le disulfure de dipropyle, le trisulfure de dipropyle, le disulfure de méthypropyle, le L-menthol, la menthone, la L-carvone, l'acétat d'isoamyle, la 2-acétyl-1-pyrroline, le 2)~,4Z-décadiénal, le 3,5-diméthyltrithiolane, le citral, le caryophyllène, l'1-octène-3-ol, l'1-octène-3-one, l'hydroxybenzylacétone, le cis-3-hexénol, le 3Z-hexénol, le butyrate de méthyle, le géraniol, l'éthyl-2E,4Z-décadiénoate, la 8-mercapto-p-menth-1-én-3-one, le 2E,4Z,7Z-tridécatriénal, le 2E,5Z-undécadiénal, le nonanal, le 4-octanolide, le 5-octanolide, le phényléthanol, la lactone des vins et la menthofurolactone,

ii. un ou plusieurs agents de sapidité et/ou matières odoriférantes qui communiquent une impression sensorielle négative, choisis parmi la liste consistant en des alcools en $C_1$ à $C_5$, de préférence le méthanol, l'éthanol, le propanol, l'isopropanol, le butanol, le 2-méthylbutanol, le 3-méthylbutanol, le diacétyle, l'acétaldéhyde, le furfural, l'alcool furfurylique, le phénol, l'acétoïne, le sulfure de diméthyle, le méthylmercaptan, l'acide lactique et l'acide acétique.

sachant que la proportion de solvant, rapportée au volume total de la solution aqueuse, se trouve dans l'intervalle de 0 % en volume à moins de 1 % en volume,

b) mise à disposition d'un matériau d'adsorption dans un dispositif approprié,

c) acheminement de la solution provenant de l'étape a) à travers le dispositif avec du matériau d'adsorption provenant de l'étape b), de telle sorte qu'aussi bien lesdits un ou plusieurs agents de sapidité et/ou matières odoriférantes provenant du groupe i) que lesdits un ou plusieurs agents de sapidité et/ou matières odoriférantes provenant du groupe ii) soient adsorbés sur le matériau d'adsorption,

d) mise à disposition d'un ou de plusieurs solvants ou de mélanges de solvants avec de l'eau, les solvants ou les mélanges de solvants étant choisis de telle sorte qu'un ou plusieurs agents de sapidité et/ou matières odoriférantes du groupe ii) présentent une valeur log pow* de 1,2 ou moins et

e) désorption des agents de sapidité et/ou des matières odoriférantes depuis le matériau d'adsorption provenant de l'étape c) avec les solvants ou les mélanges de solvants provenant de l'étape d), lors de quoi une, deux, trois, quatre, cinq, six, sept fractions ou plus sont collectées en fonction du temps et lors de quoi, dans une de ces fractions ou dans un mélange de deux, trois, quatre, cinq, six, sept de ces fractions ou plus, lesdits un ou plusieurs agents de sapidité et/ou matières odoriférantes provenant du groupe i), se retrouvent plus enrichis d'un facteur de 1,5 ou plus respectivement, en comparaison avec les un ou plusieurs agents de sapidité et/ou matières odoriférantes provenant du groupe ii), indépendamment les uns des autres, que dans l'arôme aqueux provenant de l'étape a), sachant que le solvant utilisé pour la désorption ou les solvants utilisés pour la désorption sont choisis de telle sorte que les agents de sapidité et/ou les matières odoriférantes à désorber possèdent une valeur log pow* de 1,20 ou moins et que la valeur log pow* est calculée comme suit :

$$\log pow^* = \log pow - x^* \, P'(x) \, / \log Pow$$

avec log pow comme logarithme décimal du coefficient de répartition de l'agent de sapidité et/ou de la matière odoriférante entre le 1-octanol et l'eau,

avec x comme la proportion résultante de solvant rapportée à la part totale en volume, normée à 1,0, et

avec P'(x) comme le paramètre de polarité pour le solvant correspondant pour la proportion résultante x du solvant.

9. Procédé selon la revendication 8 pour la préparation d'un concentré d'arôme, dans lequel un ou plusieurs agents de sapidité et/ou matières odoriférantes qui communiquent une impression sensorielle positive, indépendamment les uns des autres, sont plus fortement enrichis d'un facteur 1,5 ou plus, respectivement, rapporté à un arôme aqueux, en comparaison avec un ou plusieurs agents de sapidité et/ou matières odoriférantes qui communiquent une impression sensorielle négative, comportant ou consistant en les étapes suivantes :

étapes a) à d) selon la revendication 8 et

e) désorption des agents de sapidité et/ou des matières odoriférantes depuis le matériau d'adsorption provenant de l'étape c) avec les solvants ou les mélanges de solvants provenant de l'étape d), lors de quoi une, deux, trois, quatre, cinq, six, sept fractions ou plus sont collectées en fonction du temps et lors de quoi une de ces fractions ou un mélange de deux, trois, quatre, cinq, six, sept de ces fractions ou plus forment le concentré d'arôme, lesdits un ou plusieurs agents de sapidité et/ou matières odoriférantes provenant du groupe i), indépendamment les uns des autres, se retrouvant, dans le concentré d'arôme résultant, plus enrichis d'un facteur de 1,5 ou plus respectivement, en comparaison avec l'un ou lesdits plusieurs agents de sapidité et/ou matières odoriférantes provenant du groupe ii), que dans l'arôme aqueux provenant de l'étape a), sachant que le solvant utilisé pour la désorption ou les solvants utilisés pour la désorption sont choisis de telle sorte que les agents de sapidité et/ou les matières odoriférantes à désorber possèdent une valeur log pow* de 1,20 ou moins et que la valeur log pow* est calculée comme suit :

$$\log \text{pow*} = \log \text{pow} - x^* \, P'(x) / \log \text{Pow}$$

avec log pow comme logarithme décimal du coefficient de répartition de l'agent de sapidité et/ou de la matière odoriférante entre l'octanol et l'eau,

avec x comme la proportion résultante de solvant rapportée à la part totale en volume, normée à 1,0, et

avec P'(x) comme le paramètre de polarité pour le solvant correspondant pour la proportion résultante x du solvant,

10. Procédé selon la revendication 9, **caractérisé en ce que** la contre-pression sur la solution de l'étape d) se trouve au moins partiellement pendant le processus de désorption à l'étape e) dans l'intervalle de 0,1 bar à 4,0 bar.

11. Procédé selon l'une quelconque des revendications 2 à 7, 9 ou 10, **caractérisé en ce que** le matériau d'adsorption est choisi parmi le groupe consistant en des polystyrènes réticulés, de préférence l'éthylvinylbenzène et le divinylbenzène, la vinylpyrrolidine et le divinylbenzène, la vinylpyridine et le divinylbenzène et le styrène et le divinylbenzène; et d'autres polymères, de préférence des polyaromates, des polystyrènes, des polyméthacrylates, des polypropylènes, des polyesters et le polytétrafluoréthylène.

12. Concentré d'arôme, pouvant être préparé par un procédé selon l'une quelconque des revendications 1 à 11, le concentré d'arôme résultant comportant lesdits un ou plusieurs agents de sapidité et/ou matières odoriférantes du groupe i) plus enrichis respectivement d'un facteur 1,5 ou plus en comparaison desdits un ou plusieurs agents de sapidité et/ou matières odoriférantes du groupe ii), indépendamment les uns des autres, que l'arôme aqueux et sachant que dans l'étape a) du procédé selon l'une quelconque des revendications 1 à 11, un arôme aqueux est mis à disposition, comprenant

i. un ou plusieurs agents de sapidité et/ou matières odoriférantes qui communiquent une impression sensorielle positive, choisis parmi la liste consistant en le butyrate d'éthyle, l'éthylméthylbutyrate-2, le méthylcapronate, le linalool, l'alpha-ionone, la bêta-ionone, la deltadécalactone, le 2E-hexénol, le 2E-hexénal, l'hexanal, la bêta-damascénone, l'octanal, la nootcatone, le p-menthènethiol-1,8, le benzaldéhyde, la gamma-décalactone, l'oxyde de linalool, le furfurylthiol-2, le 4-vinylgaïacol, l'isopropylméthoxypyrazine isomère, l'éthyldiméthylpyrazine isomère, l'indol, le méthyljasmonate, la jasminolactone, le disulfure de dipropyle, le trisulfure de dipropyle, le disulfure de méthypropyle, le L-menthol, la menthone, la L-carvone, l'acétate d'isoamyle, la 2-acétyl-1-pyrroline, le 2E,4Z-décadiénal, le 3,5-diméthyltrithiolane, le citral, le caryophyllène, l'1-octène-3-ol, l'1-octène-3-one, l'hydroxybenzylacétone, le cis-3-hexénol, le 3Z-hexénol, le butyrate de méthyle, le géraniol, l'éthyl-2E,4Z-décadiénoate, la 8-mercapto-p-menth-1-én-3-one, le 2E,4Z,7Z-tridécatriénal, le 2E,5Z-undécadiénal, le nonanal, le 4-octanolide, le 5-octanolide, le phényléthanol, la lactone des vins et la menthofurolactone,

ii. un ou plusieurs agents de sapidité et/ou matières odoriférantes qui communiquent une impression sensorielle négative, choisis parmi la liste consistant en des alcools en $C_1$ à $C_5$, de préférence le méthanol, l'éthanol, le propanol, l'isopropanol, le butanol, le 2-méthylbutatzol, le 3-méthylbutanol, le diacétyle, l'acétaldéhyde, le furfural, l'alcool furfurylique, le phénol, l'acétoïne, le méthylmercaptan et l'acide lactique.

13. Produits alimentaires, stimulants, boissons, semi-finis, produits d'hygiène buccale, produits cosmétiques ou pharmaceutiques comprenant un ou plusieurs concentrés d'arôme selon la revendication 12, ainsi qu'un ou plusieurs produits de base, auxiliaires ou additifs.

14. Utilisation de concentrés d'arôme selon la revendication 12 dans des produits alimentaires, stimulants, boissons, semi-finis, des produits d'hygiène buccale, des produits cosmétiques ou pharmaceutiques.

15. Utilisation de concentrés d'arôme selon la revendication 12 pour la préparation de produits alimentaires, de stimulants, de boissons, de produits semi-finis, de produits d'hygiène buccale, de produits cosmétiques ou de produits pharmaceutiques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19835542 A1
- EP 0242325 A
- US 4518615 A
- US 5093136 A
- US 5266336 A
- US 5601858 A
- US 6986709 B
- WO 2004000787 A
- WO 2004043906 A
- WO 2005044778 A
- WO 03106404 A
- WO 2006003210 A
- US 5045336 A
- US 5643941 A
- GB 2380936 A
- US 5580545 A
- US 20020177576 A
- EP 635218 A
- US 4154862 A
- EP 1258200 A
- WO 2005096841 A
- US 3159585 A
- US 3971852 A
- US 4532145 A
- US 5124162 A

**In der Beschreibung aufgeführte Nicht-Patentliteratur**

- **Günter J. Eppert.** Flüssigkeitschromatographie HPLC - Theorie und Praxis. Friedr. Vieweg & Sohn Verlagsgesellschaft mbH, 1997, 84
- *Manufacturing Chemist,* 2000, 16-17
- *Nature,* 1997, vol. 387, 563